# EUROPÄISCHE PATENTSCHRIFT

(11) **EP 3 839 578 B1**
(45) Veröffentlichungstag und Bekanntmachung des Hinweises auf die Patenterteilung: **06.08.2025**
(21) Anmeldenummer: 19217565.1
(22) Anmeldetag: 18.12.2019
(51) Int. Cl.: G01T 1/24

(54) **PHOTONENZÄHLENDER RÖNTGENDETEKTOR UND VERFAHREN ZUM BETREIBEN EINES PHOTONENZÄHLENDEN RÖNTGENDETEKTORS**
PHOTON COUNTING X-RAY DETECTOR AND METHOD FOR OPERATING A PHOTON COUNTING X-RAY DETECTOR
DÉTECTEUR DE RAYONS X À COMPTAGE PHOTONIQUE ET PROCÉDÉ DE FONCTIONNEMENT D'UN DÉTECTEUR DE RAYONS X À COMPTAGE PHOTONIQUE

(43) Veröffentlichungstag der Anmeldung: 23.06.2021
(73) Patentinhaber: Siemens Healthineers AG, 91301 Forchheim (DE)
(72) Erfinder: Göderer, Edgar, 91301 Forchheim (DE); Kreisler, Björn, 91353 Hausen (DE); Hupfer, Martin, 91052 Erlangen (DE); Petersilka, Martin, 91325 Adelsdorf (DE)
(74) Vertreter: Siemens Healthineers Patent Attorneys

(56) Entgegenhaltungen:
- WO-A1-2017/144474
- WO-A2-2012/150489
- DE-A1- 102012 224 209
- DE-A1- 102015 215 086
- US-B2- 8 772 730

## Beschreibung

Die Erfindung betrifft einen photonenzählender Röntgendetektor zur Aufnahme eines Röntgenbilddatensatzes, ein medizinisches Bildgebungsgerät aufweisend einen photonenzählenden Röntgendetektor und ein Verfahren zum Betreiben des photonenzählenden Röntgendetektors.

Photonenzählende Röntgendetektoren finden in vielen bildgebenden Anwendungen Einsatz. So werden diese Röntgendetektoren beispielsweise in Computertomographen in der medizinischen Bildgebung genutzt, um ein tomographisches Röntgenbild eines Untersuchungsbereiches eines Patienten zu erzeugen.

Als photonenzählendender Röntgendetektor kann insbesondere ein photonenzählendender, direkt-konvertierender Röntgendetektoren eingesetzt werden. Eintreffende Röntgenstrahlung bzw. Photonen können in solchen Röntgendetektoren durch ein geeignetes Konvertermaterial in elektrische Pulse umgewandelt werden. Die elektrischen Pulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung dann durch Zählen der elektrischen Pulse, welche durch die Absorption von Röntgenphotonen im Konvertermaterial ausgelöst werden, gemessen. Die Höhe oder auch die Länge eines erzeugten elektrischen Pulses ist in der Regel außerdem proportional zur Energie des absorbierten Röntgenphotons. Dadurch kann eine spektrale Information durch den Vergleich der Höhe bzw. Länge des elektrischen Pulses mit einer Energieschwelle extrahiert werden. Häufig weisen photonenzählende Röntgendetektor mehrere einstellbare Energieschwellen für einen Vergleich bereit, so dass energieaufgelöste Messungen in Abhängigkeit von mehreren durch die energieschwellen definierten Energiebereiche ermöglicht sind.

Die Nutzung photonenzählender Detektoren in der Röntgenbildgebung bietet eine Reihe von Vorteilen gegenüber energieintegrierenden Detektoren. So ermöglichen sie eine hohe Ortsauflösung und eine intrinsisch energieaufgelöste Messung.

Die Verarbeitung der im Konverterelement generierten elektrischen Signale startet üblicherweise zunächst mit einer elektrischen Signal-Verstärkung und -Formung. Je nach Ausgestaltung werden hierbei außerdem die Signale mehrerer benachbarter Pixel kombiniert (genannt "Charge Summing", deutsch: Ladungssummieren). Der Pegel der entstehenden Signale wird üblicherweise anschließend mittels einem oder mehrerer Komparatoren mit einem oder mehreren Schwellwerten verglichen. Das digitalisierte Signal kann weiter folgend unterschiedlich verarbeitet werden: Es kann beispielsweise die Zahl der Schwellüberschreitungen des Komparators gezählt werden, z.B. durch Zählung einer steigenden Taktflanke mittels eines sogenannten "rising edge counters" (dt.: steigende Taktflanken-Zähler), es können die Komparatorsignale mehrere benachbarte Pixel verrechnet werden, z.B. mittels Gating oder einer Koinzidenzlogik, es können Zählwerte benachbarter Zähler verrechnet werden, z.B. mittels Fusing (Fusionierung), Summing (Summierung), es können Zeitpunkt ("Time-of-Arrival") und/oder Dauer ("Time-over-Threshold")des Komparatorsignale bestimmt werden und vieles mehr.

Typischerweise sind jedoch von diesen Verfahren nur eines oder zwei tatsächlich im ASIC implementiert und die Abfolge im Rahmen des Designs festgelegt. Die pixelweise Schaltung wird außerdem üblicherweise für jeden Pixel identisch ausgeführt und zusätzlich gegebenenfalls eine pixelübergreifende Kommunikations-Logik eingebaut, mit der die gewonnenen Informationen aus dem ASIC ausgelesen werden können.

Im Dokument US 8 772 730 B2 ist ein photonenzählender Detektor offenbart mit NxN Ausleseschaltkreisen, wobei zumindest ein Ausleseschaltkreis eine Vergleichseinheit mit einer Logikschaltung umfassen kann, die so konfiguriert ist, dass sie eine logische Operation unter Verwendung einer Mehrzahl von Vergleichsergebnissen mit einem n-ten Schwellenwert durchführt, wobei die Mehrzahl von Vergleichsergebnissen auf einem Vergleich eines vom Sensor umgewandelten elektrischen Signals mit dem n-ten Schwellenwert in jedem der NxN Ausleseschaltkreisen basiert, und das Ergebnis der logischen Operation an einen gemeinsamen Zähler ausgegeben wird.

DE 10 2012 224209 A1 offenbart einen zählenden digitalen Röntgendetektor mit einer Matrix an zählenden Pixelelementen, wobei zumindest ein Teil der zählenden Pixelelemente eine erste Schaltung aufweist, welche ausgebildet ist, das in dem jeweiligen Pixelelement direkt eingegangene Signal einzeln in ein Zählsignal zu wandeln und zu zählen, und eine zweite Schaltung aufweist, welche ausgebildet ist, das in dem jeweiligen Pixelelement direkt eingegangene Signal gemeinsam mit koinzidierend auftretenden Signalen mindestens eines benachbarten Pixelelements in ein Zählsignal zu wandeln und zu zählen, und wobei bei der Aufnahme eines Röntgenbildes mit dem zählenden digitalen Röntgendetektoren abhängig von einem oder mehreren Parametern des Röntgensystems, dem der Röntgendetektor zugeordnet ist, für jedes Pixelelement die für die Bildqualität günstigste Variante (erste oder zweite Schaltung oder beide Schaltungen) aktiviert werden kann.

Dokument WO 2017/ 144474 A1 offenbart eine Vorrichtung für die Bildgebung eines Objekts, wobei ein Röntgendetektor der Vorrichtung in Pixelgruppen unterteilt werden kann und den Pixelgruppen unterschiedliche Bereiche der Detektionsfläche des Röntgendetektors zugeordnet werden können.

Im Dokument DE 10 2015 215 086 A1 ist ein Röntgendetektor aufweisend eine Anordnung von Detektorelementen und eine Auswertelogik der Anordnung von Detektorelementen offenbart, wobei die Auswertelogikweist einen mehrstufigen Multiplexer aufweist.

Aufgabe der Erfindung ist es einen verbesserten Röntgendetektor für einen flexiblen Einsatz bereitzustellen.

Die Aufgabe wird gelöst durch die Merkmale der unabhängigen Patentansprüche. Weitere vorteilhafte und teils für sich erfinderische Ausführungsformen und Weiterbildungen der Erfindung sind in den Unteransprüchen und der nachfolgenden Beschreibung dargelegt.

Die Erfindung betrifft einen photonenzählenden Röntgendetektor gemäß Anspruch 1.

Der im Rahmen der Erfindung verwendete photonenzählende Röntgendetektor kann auch als direkt-konvertierender Röntgendetektor bezeichnet werden. Direkt konvertierende Röntgendetektoren werden meist in einem Stapelaufbau realisiert, bei dem an eine Lage des Konvertermaterials, d.h. an das Konverterelement, unterseitig eine zugeordnete Auswerteeinheit angebunden ist. Die Unterseite des Konverterelements weist üblicherweise matrixförmig eine Mehrzahl von Elektroden, im Folgenden auch Sensor-Pixelelektroden genannt, in Form von metallisierten Kontaktelementen auf. Mit diesen ist die Auswerteeinheit signalübertragungstechnisch kontaktiert, beispielsweise verlötet. Üblicherweise steht dabei einem konverterseitigen Kontaktelement jeweils ein pixelförmig ausgeführtes Gegenkontaktelement, im Folgenden auch Pixelelektrode genannt, auf Seiten der Auswerteeinheit entgegen. Die Auswerteeinheit stellt dann üblicherweise pixelweise Pixelelektroniken für die pixelweise Verarbeitung eines über die Pixelelektroden eingegangenen Signals bereit. Eintreffende Röntgenstrahlung wird im Konvertermaterial des Konverterelements in Abhängigkeit der lokal deponierten Energie eines Röntgenphotons in Ladungsträger konvertiert, basierend auf welchen in der pixelweise Pixelelektronik ein Signal, üblicherweise ein elektrischer Puls, erzeugt wird, welcher weiterverarbeitet wird. Einer pixelweisen Pixelelektronik kann ein entsprechendes Detektionsvolumen im Konverterelement zugeordnet werden, welches im Wesentlichen durch das elektrische Feld zwischen einer jeweiligen Sensor-Pixelelektrode und einer Top-Elektrode, welche auf der gegenüberliegenden Seite des Konverterelements aufgebracht ist, ausgebildet ist und welches das sensitiven Detektionsvolumen eines Pixelelements bildet.

Erfindungsgemäß weist jedes Pixelelement der Vielzahl an Pixelelementen des erfindungsgemäßen photonenzählenden Röntgendetektors eine erste, pixelweise Signalverarbeitungsstufe auf, welche im Wesentlichen eine analoge Verarbeitung des über eine Pixelelektrode eines Pixelelements eingegangenen Signals bereitstellt. Insbesondere wird das eingegangene Signal mittels des erfindungsgemäßen Signalverstärkers der ersten Signalverarbeitungsstufe verstärkt und das verstärkte Signal mittels des zumindest einen Komparators mit zumindest einem Schwellwert repräsentierend eine Energieschwelle verglichen. In dem Falle, dass das Signal den Schwellwert übersteigt, wird ein Ausgangsignal am Komparator ausgegeben. Der Übergang zu einem digitalen Signal (oder mehreren digitalen Signalen) erfolgt somit im Wesentlichen in diesem Schritt, so dass an einem jeweiligen Signalausgang der ersten Signalverarbeitungsstufe ein digitales Pixelsignal zur Weiterverarbeitung in der zweiten Signalverarbeitungsstufe bereitgestellt ist.

Die erste Signalverarbeitungsstufe eines Pixelelements der Vielzahl an Pixelelementen kann auch mehr als einen Komparator mit jeweils einem Schwellwert aufweisen, so dass mehrere digitale Pixelsingale in Abhängigkeit der Schwellwerte an mehreren Signalausgängen der ersten Signalverarbeitungsstufe eines Pixelelements der Vielzahl an Pixelelementen bereitgestellt sind.

Erfindungsgemäß weist zumindest eine Gruppe von Pixelelementen der Vielzahl an Pixelelementen des photonenzählenden Röntgendetektors eine gemeinsame zweite Signalverarbeitungsstufe auf. In einer Gruppe ist jeweils eine ausgewählte Teilzahl der Vielzahl an Pixelelementen zusammengefasst. Die zweite gemeinsame Signalverarbeitungsstufe wird folglich gemeinsam für zumindest die Pixelelemente der Gruppe bereitgestellt. Es können alle Pixelelemente der Vielzahl mit einer gemeinsamen Verarbeitungsstufe verschaltet sein. Das heißt, die zumindest eine Gruppe kann die gesamte Vielzahl umfassen. Die Vielzahl an Pixelelementen kann jedoch auch in mehrere Gruppen unterteilt sein, wobei jede Gruppe jeweils mit einer gemeinsamen zweite Verarbeitungsstufe verschaltet ist.

Die in der zweiten Signalverarbeitungsstufe bereitgestellte Mehrzahl an digitalen Logikelementen können dann als bereitgestellte gemeinsame Ressourcen für die digitale Signalverarbeitung der digitalen Pixelsignale verstanden werden, welche für die Signalverarbeitung der digitalen Pixelsignale der Pixelelemente der Gruppe der Vielzahl an Pixelelementen gemeinsam nutzbar ist. Ein digitales Logikelement kann dabei insbesondere als ein elektronischer Schaltkreis verstanden werden, welcher aus einem oder mehreren digital repräsentierten Eingangssignalen sowie aus einem Satz von Konfigurations- oder Steuersignalen eine oder mehrere Ausgangsgrößen ermittelt. Dabei kann die Schaltung auch interne Speicherelemente besitzen, welche Auswirkung auf die Verarbeitung der Eingangssignale (oder die Werte der Speicherelemente) haben. Die Arbeitsweise der Schaltung kann dabei getaktet, kombinatorisch oder ereignisgetrieben erfolgen.

Die zweite Signalverarbeitungsstufe umfasst eine konfigurierbare Schaltmatrix, welche für die Verschaltung der einer zweiten Signalverarbeitungsstufe zugeordneten Signalausgänge konfigurierbar ist, so dass für jeden zugeordneten Signalausgang basierend auf einer Auswahl der bereitgestellten digitalen Logikelemente eine Verarbeitungskette des mittels des Signalausgangs ausgegebenen digitalen Pixelsignals bereitstellbar ist. Eine Verarbeitungskette stellt insbesondere die Ver- und Hintereinanderschaltung der digitalen Logikelemente und eines jeweiligen Signalausgangs dar, so dass durch die Verschaltung die Verarbeitung eines an dem Signalausgang ausgegebenen Signals vergebenen ist. Am Ende der Verarbeitungskette kann dann je nach Verschaltung ein oder auch mehrere verarbeitete digitale Signale bereitgestellt werden. Die konfigurierbare Schaltmatrix ermöglicht also eine konfigurierbare Verschaltung der Signalausgänge mit zumindest einer Auswahl der bereitgestellten Logikelementen. Ebenso kann die Schaltmatrix eine konfigurierbare Verschaltung der Logikelemente untereinander ermöglichen.

Die Auswahl an digitalen Logikelementen umfasst zumindest eine Teilzahl der bereitgestellten digitalen Logikelemente.

Die Logikelemente können jeweils mit einem oder mehreren Signalausgängen der ersten Signalverarbeitungsstufe und/oder jeweils wieder mit einem oder mehreren digitalen Logikelementen verschaltet sein.

Das oder die verarbeiteten digitalen Pixelsignale können, müssen aber nicht notwendigerweise, einem konkreten Pixelelement zuordenbar sein. Das oder die verarbeiteten Pixelsignale können auch jeweils auf einer Mehrzahl an digitalen Pixelsignalen verschiedener Signalausgänge und damit auch verschiedener Pixelelemente der Gruppe basieren. Beispielsweise können die digitale Pixelsignale verschiedener Signalausgänge miteinander verrechnet werden, beispielsweise mittels einer Koinzidenzlogik oder einer Summationslogik.

Die Schaltmatrix kann dabei ähnlich wie ein FPGA ("fieldprogrammable gate array", übersetzbar in "im Feld programmierbare (Logik-)Gatter-Anordnung") ausgebildet sein. Das heißt die Schaltmatrix kann als integrierter Schaltkreis, in welchen eine logische Schaltung geladen werden kann, ausgebildet sein, wobei durch eine Programmierung bzw. Konfiguration der Schaltmatrix die gewünschte Schaltungsstruktur definierbar, d.h. anpassbar, ist. Dadurch können durch die Konfiguration der intern vorhandenen digitalen Logikelemente verschiedene Schaltungen und Funktionen realisiert werden und insbesondere wiederholt umkonfiguriert werden. Die Schaltmatrix kann insbesondere nach Auslieferung des Röntgendetektors, während der Laufzeit und für unterschiedliche Messungen und Anwendungen neu konfiguriert werden. Dadurch kann die Signalverarbeitungskette der digitalen Pixelsignale und damit die Funktionen des Röntgendetektors anpassbar und konfigurierbar bereitgestellt werden.

Dabei ist für jeden mit der gemeinsamen zweiten Signalverarbeitungsstufe verschalteter Signalausgang eine Verarbeitungskette zumindest bereitstellbar. Es kann jedoch auch Konfigurationen der Schaltmatrix geben, in denen eine Verarbeitung ausgewählter Signalausgänge nicht vorgesehen ist, d.h. bei denen nach einer Konfiguration der Schaltmatrix entsprechend keine Verarbeitungskette bereitgestellt wird. Dies ist insbesondere der Fall, wenn auf bestimmte Pixelsignale zu Gunsten beispielsweise geringerer Datenraten, einem geringeren Leistungsverbrauch oder ähnlichem verzichtet werden soll. Das heißt, es kann nach einer Konfiguration der Schaltmatrix jeweils einer digitale Verarbeitungskette für zumindest einen Teil oder auch alle der Signalausgänge der ersten Signalverarbeitungsstufe der zumindest einen Gruppe von Pixelelementen bereitgestellt werden.

Es kann bevorzugt für jeden mit einer gemeinsamen zweiten Signalverarbeitungsstufe verknüpften Signalausgang jeweils individuell eine Verarbeitungskette an digitalen Logikelementen konfigurierbar sein. Es kann jedoch auch Ausführungsvarianten des photonenzählenden Röntgendetektors geben, bei denen die Signalverarbeitungsketten für die Signalausgänge der Pixelelemente einer Gruppe jeweils nur gemeinsam konfigurierbar sind, so dass für jedes Pixelelement jeweils identische Verarbeitungsketten für die bereitgestellten digitalen Pixelsignale eines Pixelelements bereitgestellt sind. Dies kann jedoch trotzdem umfassen, dass für unterschiedliche Pixelsignale eines Pixelelements unterschiedliche Signalverarbeitungsketten an Logikelementen bereitgestellt werden.

Die erste Signalverarbeitungsstufe ist als durch pixelweise Pixelelektroniken ausgebildet zu verstehen. Einem erfindungsgemäßen Pixelelement der Vielzahl an Pixelelementen ist dann jeweils eine erste Signalverarbeitungsstufe, eine entsprechende Pixel- bzw. Sensorpixelelektrode und ein Detektionsvolumen im Konverterelement zugeordnet. Dagegen kann die zweite Signalverarbeitungsstufe zwar jeweils die digitale Verarbeitungskette der pixelweisen Pixelsignale bereitstellen, jedoch als Ganzes nicht einem einzelnen konkreten Pixelelement, sondern lediglich einer Gruppe an Pixelelementen, zugeordnet werden.

Die erfindungsgemäße Implementierung des Röntgendetektors ermöglicht vorteilhaft, die analoge und die digitale Signalverarbeitung verstärkt zu entkoppeln. Vorteilhaft kann die analoge Signalverarbeitung (Verstärkung und Komparator) möglichst sensornah durchgeführt werden und die digitale Weiterverarbeitung deutlich abstrahiert und für eine Pixelgruppe gemeinsam angelegt werden.

Die Abstraktion der digitalen Signalverarbeitung ermöglicht eine hohe Flexibilität und Wiederverwendbarkeit der Auswerteeinheit. Eine Zusammenlegung von Pixelgruppen mittels einem konfigurierbaren Matrixreadout, d.h. eine zweite Signalverarbeitungsstufe mit einer konfigurierbaren Schaltmatrix, ermöglicht vorteilhaft eine messungsangepasste Verarbeitung der Pixelsignale. Vorteilhaft kann durch einen Anwender die Verschaltung der bereitgestellten Logikelemente konfiguriert und angepasst werden.

Indem ein jeweiliges sensitives Detektionsvolumen eines Pixelelement direkt an den analogen Teil der Signalvorverarbeitung angeschlossen ist, kann eine analoge Signalvorverarbeitung mit minimaler Impedanz, geringem Rauschen und geringer Leistungsaufnahme ermöglicht werden. Das verstärkte analoge Ausgangssignal des Signalverstärkers ist dabei bevorzugt unmittelbar mit dem zumindest einem oder mehreren Komparatoren verbunden. Dies ermöglich hohe Schaltfrequenzen und präzises Timing und produziert ein digitales Pixelsignal, welches robust gegenüber der Weiterleitung an die über die Schaltmatrix signaltechnisch verbundenen digitalen Logikelementen ist.

Durch die erfindungsgemäße Architektur kann also eine besonders vorteilhafte Analogperformance einer sensornahen Signalverarbeitung und Digitalisierung mit der Flexibilität einer hochgradig konfigurierbaren digitalen Verarbeitungskette kombiniert werden.

Die erfindungsgemäße Implementierung des Röntgendetektors ermöglicht außerdem eine ressourceneffiziente Nutzbarkeit der Auswerteeinheit auch beispielsweiße bei großem Pixel-Pitch (z.B. Bereitstellung einer elektrisch leitenden Verbindung zwischen Auswerteeinheit und Konverterelement nur jede 2. Pixelelektrode). Konkret können diejenigen Pixelelektroden und die damit gekoppelten entsprechenden ersten Signalverarbeitungsstufen, die über die elektrisch leitende Verbindung nicht mit einer Sensorpixelelektrode verbunden sind, einfach deaktiviert werden bzw. in der Schaltmatrix ungenutzt bleiben. Die benutzen Pixel hingegen können nach wie vor, z.B. in einer Koinzidenzlogik mit ihren tatsächlichen Nachbarn, verbunden werden.

Es kann dazu vorteilhaft zweckmäßig vorgesehen sein, dass der erfindungsgemäße Röntgendetektor bzw. die zumindest eine Schaltmatrix über einstellbare Registerparameter konfigurierbar ist, so dass in Abhängigkeit der eingestellten Registerparameter unterschiedliche digitale Verarbeitungsketten für die digitalen Pixel-Signale bereitstellbar sind.

Die Registerparameter können beispielsweise über eine SteuerSchnittstelle des Röntgendetektors anpassbar, d.h. im Wesentlichen programmierbar, sein. Derart kann die Logik der Schaltmatrix durch die Anpassung der Registerparameterwerte für unterschiedliche Messungen und/oder Anwendungen neu parametriert und damit angepasst werden.

Dadurch kann vorteilhaft in einfacher Weise auch eine automatische Parametrierung ermöglicht werden. Beispielsweise können extern in einer Speichereinheit oder auch in mehreren Registern der Schaltmatrix mehrere Sätze an Registerparameterwerten vorgehalten werden.

Ein Satz an Registerparameterwerten kann jeweils in Abhängigkeit einer Röntgenanwendung vorliegen. Die Wahl einer bestimmten Röntgenanwendung, beispielsweise hinsichtlich eines zu untersuchenden Objekts, eines bestimmten Aufnahmebereichs, eines Anwendungsablaufs oder einer bestimmten Aufnahmemodalität, lässt zu einem großen Teil Rückschlüsse über die Aufnahmebedingungen während der Aufnahme des Röntgenbilddatensatzes, einer gewünschten Bildqualität und/oder einer zu erzielenden Bildinformation zu und erlaubt damit vorteilhaft eine bedarfsgerechte Anpassung des Röntgendetektors.

Die Sätze an Registerparameterwerten können auch beispielsweise in Verknüpfung mit einem oder mehreren Anwendungsparametern vorliegen. Ein Anwendungsparameter kann ein Parameter der Röntgenanwendung und/oder des medizinischen Bildgebungsgeräts dem der erfindungsgemäße Röntgendetektor zugeordnet und/oder mit dem er baulich verbunden ist sein.

Da heißt, ein Anwendungsparameter kann direkt mit einer Röntgenanwendung verknüpft oder aus von ihr ableitbar sein. Beispielsweise kann der Anwendungsparameter auf einem Röntgenfluss, einem Zeitpunkt oder einer Zeitdauer, einem Steuerungsparameter des medizinischen Röntgengeräts, beispielsweise ein Röntgenröhrenstrom oder einer Röntgenröhrenspannung, einem Bildparameter des aufzunehmenden Röntgenbilddatensatzes, beispielsweise einer spektralen Auflösung oder einer Ortsauflösung, basieren.

Ein Anwendungsparameter kann außerdem auch beispielsweise durch das medizinische Bildgebungsgerät, dem der erfindungsgemäße Röntgendetektor zugeordnet ist, vorgegebenen Randbedingungen basieren, beispielsweise eine zur Verfügung stehende Kühlleistung oder einer Leistungsversorgung.

Vorteilhaft ist eine einfache und gegebenenfalls automatische Konfiguration des Röntgendetektors möglich, so dass stets die für eine Röntgenanwendung, die gegebenen Randbedingungen eines medizinischen Röntgengeräts und/oder eine Messung günstigste Konfiguration des Röntgendetektors bereitstellbar ist.

In einer weiteren Ausführungsform des photonenzählender Röntgendetektors ist für eine erste Anzahl der Signalausgänge der Gruppe von Pixelelementen eine erste Verarbeitungskette und für eine zweite Anzahl an Signalausgängen eine von der ersten Verarbeitungskette verschiedene zweite Verarbeitungskette bereitstellbar.

Vorteilhaft ist eine flexible Konfiguration und bedarfsgerechte Anpassung der Signalverarbeitung möglich.

Dabei können für unterschiedliche Signalausgänge eines Pixelelements unterschiedliche Verarbeitungsketten bereitgestellt werden. Beispielsweise können je nach Bedarf für einen Teil der Signalausgänge eines Pixelelements eine Verschaltung zu Koinzidenzlogiken oder zu einer Summenschaltung vorgesehen sein. Beispielsweise kann dadurch eine unabhängige Konfiguration von räumlicher und spektraler Auflösung ermöglicht werden, indem teilweise digitale Pixelsignale eines Pixelelements in voller Ortsauflösung übertragen werden können, wohingegen ein anderer Teil der digitalen Pixelsignale des Pixelelements mit Pixelsignalen anderer Pixelelemente verrechnet werden können.

Ebenso können für die digitalen Pixelsignale von Pixelelementen, welche unterschiedlichen Randbedingungen unterliegen, jeweils unterschiedliche Signalverarbeitungsketten konfiguriert werden. Beispielsweise können Pixelelemente, welche randseitig innerhalb der matrixartigen Anordnung der Pixelelemente angeordnet sind und damit weniger direkt benachbarte Pixelelemente aufweisen, anderweitig konfiguriert werden als Pixelelemente, welche mittig angeordnet sind.

Ferner umfasst die Mehrzahl an digitalen Logikelemente zumindest ein Zählelement und ein Ausleseelement.

Dadurch kann in einer einfachsten Konfiguration des Röntgendetektors ein einfacher photonenzählender Röntgendetektor bereitgestellt werden.

Das Zählelement umfasst eine Mehrzahl an Zählern.

Ein Zähler ist ausgebildet basierend auf einem eingehenden Signal einen Zählerstand des Zählers um eine Zähleinheit zu erhöhen.

Das Zählelement kann also einem Ressourcenblock aufweisend eine Mehrzahl an Zählern entsprechen, so dass basierend auf mehreren Signalausgängen jeweils eine Anzahl an erzeugten digitalen Pixelsignalen gezählt und zumindest zeitweise gespeichert werden kann.

Statt beispielsweise 4 Zählern pro Pixelelement kann beispielsweise ein konfigurierbarer Ressourcenblock, d.h. ein Zählelement, mit 48 Zählern bereitgestellt sein, der von einer Gruppe von beispielsweise 16 Pixelelementen gemeinsam konfigurierbar genutzt werden kann.

Mittels des Ausleseelements können dann die Zählerstände des Zählelements ausgelesen werden, so dass darauf basierend ein Röntgenbilddatensatz erzeugt werden kann.

Gemäß einer weiteren bevorzugten Ausgestaltungsform des Photonenzählender Röntgendetektor umfasst die Mehrzahl an digitalen Logikelementen außerdem zumindest ein Element der folgenden Liste
- eine Koinzidenzlogik,
- ein Signalverzögerungselement,
- ein Pufferelement,
- ein Schaltelement zur Verhinderung einer Paralyse eines Zählelements,
- ein Kombinatorisches Logikgatter,
- ein Multiplexer,
- ein Registerelement,
- ein Element, welches bei Auslösen einen Puls fester oder konfigurierbarer Länge erzeugt, oder
- ein Element, welches den Zeitpunkt oder die Zeitdauer eines Triggers misst.

Vorteilhaft können bevorzugte Verschaltungen des Röntgendetektors realisiert werden. Insbesondere umfasst die Mehrzahl an digitalen Logikelementen eine Kombination von Elementen der Liste. Darüber hinaus können auch noch andere Arten von digitalen Logikelementen vorgesehen sein.

Weiterhin kann vorgesehen sein, dass ein digitales Logikelement der Mehrzahl an digitalen Logikelementen in der zweiten Signalverarbeitungsstufe mehrfach bereitgestellt ist.

Die jeweiligen digitalen Logikelemente kann es je nach Einsatzzweck und nach verfügbarem Platz und verfügbarer Leistung in unterschiedlicher Auswahl und Anzahl geben. Vorteilhaft zweckmäßig können für die Signalausgänge geeignete Signalverarbeitungsketten bereitgestellt werden.

In einer weiteren Ausführungsform des erfindungsgemäßen photonenzählenden Röntgendetektors sind nach einer Konfiguration der konfigurierbaren Schaltmatrix nicht verschaltete digitale Logikelemente der Mehrzahl an digitalen Logikelementen von einer Leistungsversorgung getrennt.

Vorteilhaft sind nicht genutzte Logikelemente abschaltbar. Beispielsweise kann dies mittels der einstellbaren Registerparameter vorgegeben und konfiguriert werden. Vorteilhaft kann ein verbrauchseffizienter Röntgendetektor jeweils angepasst an die Anwendung und die Randbedingungen bereitgestellt werden. Ferner kann die gleiche Auswerteeinheit durch gezielt sparsames Nutzen der Ressourcen auch für Anwendungen/Produkte eingesetzt werden, in denen weniger Leistung-/Kühlbudget zur Verfügung steht. Alternativ kann die einsetzbare Leistungsfähigkeit und der Funktionsumfang der Auswerteeinheit durch Optimierung des Kühlbudgets in einfacher Weise und auch nachträglich erhöht werden. Die Erfindung ermöglicht damit vorteilhaft die Nutzbarkeit der gleichen Auswerteeinheit in unterschiedlichen Produkten und/oder Anwendungen und damit einen flexiblen Einsatz.

Gemäß einer weiteren Ausgestaltungsvariante des photonenzählenden Röntgendetektors ist die Vielzahl an Pixelelemente in eine Mehrzahl an Gruppen von Pixelelementen unterteilt, wobei jeder Gruppe jeweils eine konfigurierbare Schaltmatrix zugeordnet ist.

Eine Gruppe kann beispielsweise jeweils einem ASIC zugeordnet sein. Es können jedoch mehrere Pixelgruppen einem ASIC zugeordnet sein. Eine Gruppe kann auch eine anderweitige Auswahl an Pixelelementen umfassen, beispielsweise kann eine Gruppe durch die Anordnung relativ zu einem Streustrahlenraster (ASG, "Anti-Scatter-Grid") definiert sein. Beispielsweise umfasst eine Gruppe jeweils eine ASG-Pixelgruppe, welche jeweils einem Durchgangskanal des ASGs zugeordnet ist. Es kann auch andere Gruppierungen geben. Beispielsweise kann eine jeweilige Gruppe jeweils eine feste Anzahl an Pixelelemente umfassen, welche zueinander in einer gleichartigen Beziehung stehen. Beispielsweise ist die Beziehung jeweils durch ihre Anordnung relativ zueinander gegeben. Beispielsweise umfasst eine Gruppe an Pixelelementen jeweils ein Verbund von 8x8 oder 16x16 Pixelelementen. Ein solcher Verbund kann auch als Makropixel bezeichnet werden, wobei Signale der Pixelelemente eines Verbunds bei Bedarf bevorzugt miteinander verrechnet werden.

Durch eine Unterteilung der Vielzahl an Pixelelementen in mehrere Gruppen an Pixelelementen, welche jeweils einer Schaltmatrix zugeordnet sind, kann vorteilhaft die Komplexität einer jeweiligen Schaltmatrix oder der Konfiguration der Schaltmatrix auf einem geringeren Niveau gehalten werden. Vorteilhaft lässt sich außerdem die Konfiguration einer Schaltmatrix einfach auf eine Vielzahl an Schaltmatrizen übertragen.

Gemäß einer dazu alternativen Ausgestaltungsvariante des photonenzählenden Röntgendetektors Photonenzählender Röntgendetektor ist die Vielzahl an Pixelelemente in eine Mehrzahl an Gruppen von Pixelelementen unterteilt, wobei der Mehrzahl an Gruppen eine gemeinsame konfigurierbare Schaltmatrix zugeordnet ist.

Vorteilhaft kann über Gruppen von Pixelelementen hinweg vereinfacht eine Verschaltung und ein Signalaustausch realisiert werden. Beispielsweise kann eine Koinzidenzbetrachtung über Pixelgruppen hinweg vereinfacht realisiert werden. Die Gruppen können beispielsweise über gemeinsame externe Parameter definiert sein. Beispielsweise können die Gruppen jeweils eine ASG-Pixelgruppe oder ein Makropixel umfassen.

In einer vorteilhaften Variante kann dabei trotzdem vorgesehen sein, dass die eine konfigurierbare Schaltmatrix gruppenbasiert konfigurierbar ist.

Beispielsweise sind in der Schaltmatrix jeweils gleichartig die gleiche Anzahl und Auswahl der digitalen Logikelemente pro Pixelgruppe vorgesehen. Beispielsweise kann die Konfiguration einer Teilmatrix der Schaltmatrix auf den Rest der Schaltmatrix gruppenbasiert übertragen werden, d.h. die Konfiguration gruppenbasiert repliziert und angewendet werden.

In einer weiteren Ausführungsform des erfindungsgemäßen photonenzählenden Röntgendetektors sieht die Auswerteeinheit zumindest einen ersten Bereich für die erste Verarbeitungsstufe und einen zweiten Bereich für die zweite Verarbeitungsstufe vor, wobei der zumindest eine erste Bereich inselförmig innerhalb des zweiten Bereich oder randseitig entlang des zweiten Bereichs vorgesehen ist.

Durch die Entkoppelung von erster, im Wesentlichen analoger Signalverarbeitungsstufe und zweiter, digitaler Signalverarbeitungsstufe eine flexiblere Anordnung und ein flexibleres Floorplanning (dt.: Grundrisserstellung; Planung der Anordnung der Teilschaltungen in der Auswerteeinheit) von Digital- und Analogteil der Signalverarbeitung. Vorteilhaft kann dadurch die flächenmäßig kleiner ausbildbare pixelspezifische Schaltung, d.h. die jeweilige erste Signalverarbeitungsstufe, außerdem kleinere Pixelelement-Abstände ermöglichen.

Konkret kann die Entkoppelung von erster Signalverarbeitungsstufe und zweiter Signalverarbeitungsstufe vorteilhaft beispielsweise ermöglichen, den für die erste Signalverarbeitungsstufe eines jeweiligen Pixelelements bereitgestellte Bereich der Auswerteeinheit jeweils inselförmig innerhalb eines Bereichs, welcher die digitale Signalverarbeitungsstufe für zumindest eine Gruppe an Pixelelementen bereitstellt, einzubetten. Ebenso kann vorgesehen sein, die die Flächenbereiche der ersten Signalverarbeitungsstufen einer Gruppe von Pixelelementen auch zu Streifen, Zentren oder Ringen zusammenzufassen. Beispielsweise können die ersten Signalverarbeitungsstufen zusammengefasst jeweils randseitig entlang des zweiten Bereichs, welcher für die zweite Signalverarbeitungsstufe vorgesehen ist, angeordnet sein. Beispielsweise können die ersten Signalverarbeitungsstufen zusammengefasst jeweils mittig innerhalb des zweiten Bereichs, welcher für die zweite Signalverarbeitungsstufe vorgesehen ist, angeordnet sein.

Gemäß einer weiteren Ausgestaltungsvariante des photonenzählenden Röntgendetektors ist die Auswerteeinheit schichtweise aufgebaut, wobei in einer ersten Schicht die erste Verarbeitungsstufe bereitgestellt ist und in einer in Strahleneinfallsrichtung hinter der ersten Schicht gelegenen, zweiten Schicht die zweite Verarbeitungsstufe bereitgestellt ist.

Vorteilhaft kann dadurch beispielsweise die flächenmäßig kleiner ausbildbare pixelspezifische erste Signalverarbeitungsstufe kleinere Pixelelement-Abstände ermöglichen.

In einer Weiterbildung davon kann eine Aufteilung der analogen und digitalen Schaltkreise, d.h. der jeweiligen ersten Verarbeitungsstufen und der zweiten Verarbeitungsstufe, in separate Wafer-Dies (separate Teilbereiche eines oder verschiedener Halbleiter-Wafer) vorgesehen sein. Bei einer solchen Aufteilung in separate Dies können diese in einem Die-Stacking Prozess (dt.: Stapelprozess) zu einer Auswerteeinheit zusammengefügt werden. Bei Bereitstellung der ersten und zweiten Signalverarbeitungsstufe in separaten Dies sind die analogen und digitalen Schaltkreise nicht mehr an die gleiche Technologie gebunden, so dass beispielsweise unterschiedliche Strukturgrößen bei der Bereitstellung genutzt werden können und somit auch unabhängig voneinander optimiert werden können. Derart können beispielsweiße außerdem unterschiedliche Pixelgrößen bei gleichbleibender Implementierung der zweiten Signalverarbeitungsstufe erreicht werden.

Der erfindungsgemäße photonenzählenden Röntgendetektor kann gemäß einer Ausführungsvariante entlang der Röntgenstrahleneinfallsrichtung zwischen dem Konverterelement und der Auswerteeinheit eine Umverdrahtungsschicht aufweisen.

Eine Umverdrahtungsschicht umfasst insbesondere elektrisch leitende Verbindungen, welche eine erste räumliche Verteilung von Eingangskontaktelementen auf eine zweite räumliche Verteilung von Ausgangskontaktelementen überträgt. Vorteilhaft zweckmäßig kann durch eine Bereitstellung einer Umverdrahtungsschicht die Position der Sensor-Pixelelektroden und die auswerteseitigen Pixelelektroden entkoppelt werden und damit eine flexible Anordnung der den Schaltungsteilen zugeordneten Bereiche, d.h. der ersten Signalverarbeitungsstufen und der zweiten Signalverarbeitungsstufe einer jeweiligen Gruppe an Pixelelementen, ermöglicht werden.

Die Erfindung betrifft außerdem ein medizinisches Bildgebungsgerät aufweisend einen erfindungsgemäßen photonenzählenden Röntgendetektor.

Die Merkmale und Vorteile des photonenzählenden Röntgendetektors können dabei direkt auf das medizinischen Bildgebungsgerät übertragen werden.

Das medizinische Bildgebungsgerät kann insbesondere als medizinisches Röntgengerät ausgebildet sein. Das medizinische Bildgebungsgerät kann insbesondere eine dem photonenzählenden Röntgendetektor zugeordnete Röntgenquelle zur Belichtung des Röntgendetektors umfassen. Üblicherweise umfasst das medizinische Bildgebungsgerät zumindest einen erfindungsgemäßen photonenzählenden Röntgendetektor und in Gegenüberstellung dazu zumindest eine Röntgenquelle, beispielsweise eine Röntgenröhre. Für die Aufnahme des Röntgenbilddatensatzes kann dann insbesondere zwischen die Röntgenquelle und den photonenzählenden Röntgendetektor ein abzubildendes Objekt platziert und mittels der Röntgenquelle durchstrahlt werden. Insbesondere kann das medizinische Bildgebungsgerät als Computertomographie-System ausgebildet sein. Es kann aber auch beispielsweise als C-Bogen-Röntgengerät und/oder Dyna-CT oder anderweitig ausgebildet sein.

Die Erfindung betrifft außerdem ein Verfahren zum Betreiben eines photonenzählenden Röntgendetektors, gemäß Anspruch 12.

Dabei ist der Röntgendetektor einer Röntgenquelle zur Ausstrahlung einer Röntgenstrahlung zugeordnet.

Durch die Möglichkeit der Anpassung der Registerparameterwerte der Registerparameter kann die Logik der Schaltmatrix für unterschiedliche Messungen und/oder Anwendungen vorteilhaft einfach und wiederholt parametriert und damit angepasst werden.

In einer Ausführungsvariante des erfindungsgemäßen Verfahrens werden in einem Schritt des zweiten Anpassens die einstellbaren Registerparameter basierend auf einem zweiten Satz an Registerparameterwerten angepasst, wobei in einem Schritt des zweiten Konfigurierens der zumindest einen konfigurierbaren Schaltmatrix mittels des zweiten Sets an Registerparameterwerten eine zweite, von der ersten verschiedene, digitale Verarbeitungskette für zumindest einen Teil der Signalausgänge der ersten Signalverarbeitungsstufe der zumindest einen Gruppe von Pixelelementen bereitgestellt wird.

Gemäß einer vorteilhaften Verfahrensvariante wird der erste und/oder zweite Satz an Registerparameterwerten in Abhängigkeit einer Röntgenanwendung bestimmt.

Die Wahl einer Röntgenanwendung, beispielsweise hinsichtlich eines zu untersuchenden Objekts, eines bestimmten Aufnahmebereichs, eines Anwendungsablaufs oder einer bestimmten Aufnahmemodalität, bestimmt zu einem großen Teil die Aufnahmebedingungen während der Aufnahme des Röntgenbilddatensatzes, ebenso wie eine gewünschte Bildqualität und/oder einer zu erzielenden Bildinformation.

Die Bestimmung der Registerparameterwerte in Abhängigkeit der Röntgenanwendung erlaubt damit vorteilhaft eine jeweils vorteilhaft bedarfsgerechte Anpassung des Röntgendetektors.

Neben den in dieser Anmeldung ausdrücklich beschriebenen Ausführungsformen der Erfindung sind vielfältige weitere Ausführungsformen der Erfindung denkbar, zu denen die Fachperson gelangen kann, ohne den Bereich der Erfindung zu verlassen, der durch die Ansprüche vorgegeben ist.

Die Verwendung der unbestimmten Artikel "ein" bzw. "eine" schließt nicht aus, dass das betroffene Merkmal auch mehrfach vorhanden sein kann.

Im Folgenden wird die Erfindung anhand von beispielhaften Ausführungsformen unter Hinweis auf die beigefügten Figuren erläutert. Die Darstellung in den Figuren ist schematisch, stark vereinfacht und nicht zwingend maßstabsgetreu. Es zeigen:
Fig. 1 schematisch eine beispielhafte Ausführungsform eines Röntgendetektors 1,
Fig. 2 ein Schema zur Veranschaulichung einer signaltechnischen Verschaltung einer Gruppe an Pixelelementen 5 mit einer ersten und einer zweiten Signalverarbeitungsstufe,
Fig. 3 ein Schema zur Veranschaulichung einer signaltechnischen Verschaltung mehrerer Gruppen an Pixelelementen 5 mit einer ersten und einer zweiten Signalverarbeitungsstufe,
Fig. 4 ein Schema zur Veranschaulichung einer signaltechnischen Verschaltung einer Gruppe an Pixelelementen 5 mit einer beispielhafte Verarbeitungskette gemäß einer ersten Konfigurationsvariante,

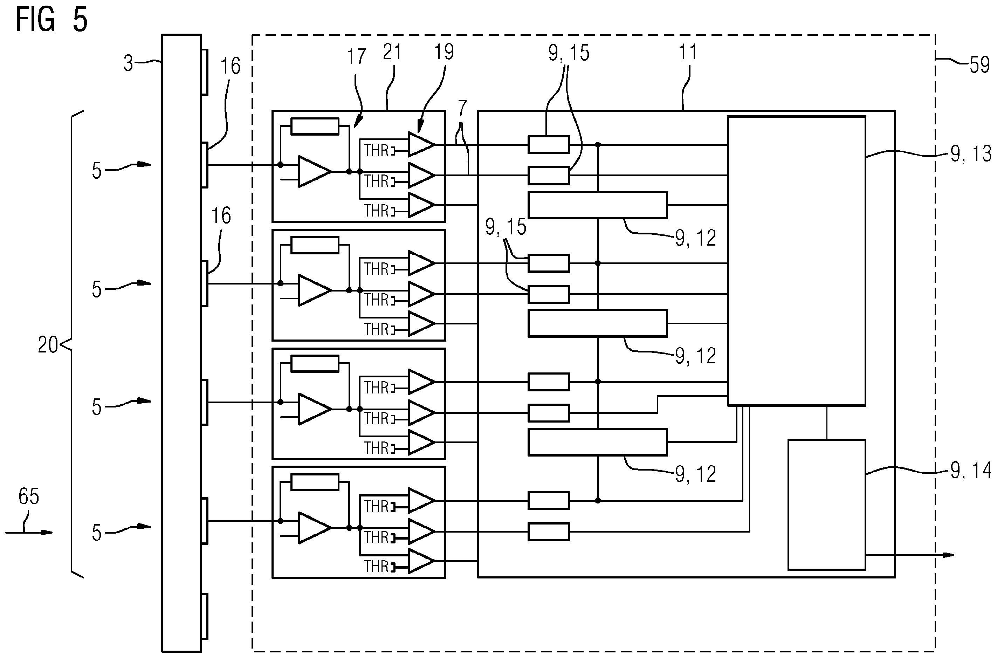

Fig. 5 ein Schema zur Veranschaulichung einer signaltechnischen Verschaltung einer Gruppe an Pixelelementen 5 mit einer beispielhafte Verarbeitungskette gemäß einer zweiten Konfigurationsvariante,
Fig. 6 ein Schema zur Veranschaulichung einer signaltechnischen Verschaltung einer Gruppe an Pixelelementen 5 mit einer beispielhafte Verarbeitungskette gemäß einer dritten Konfigurationsvariante,
Fig. 7 bis Fig. 10 jeweils eine Veranschaulichung einer beispielhaften relativen Anordnung der für eine erste und eine zweite Verarbeitungsstufe vorgesehenen Bereiche einer Auswerteeinheit,
Fig. 11 ein beispielhaftes medizinisches Bildgebungsgerät, und
Fig. 12 einen schematischen Verfahrensablauf eines Verfahrens zum Betreiben eines photonenzählenden Röntgendetektors zur Erzeugung eines Röntgenbilddatensatzes.

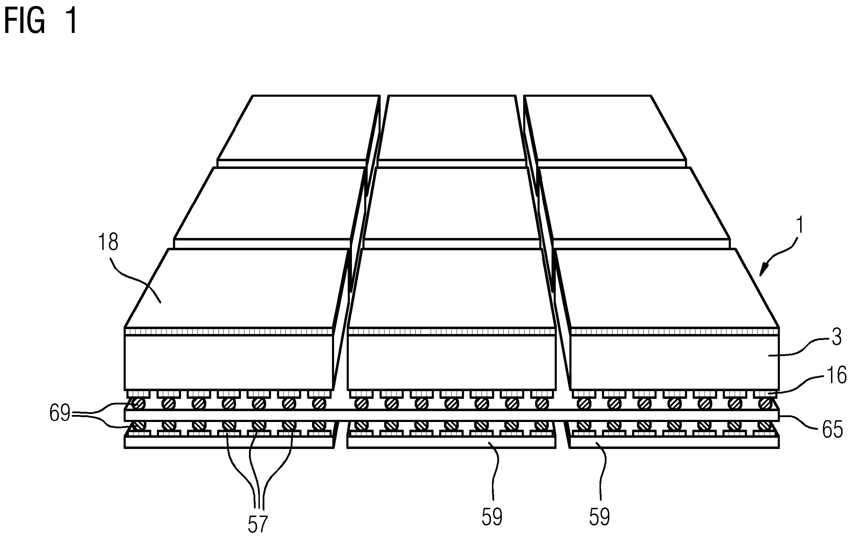

Die Fig. 1 zeigt schematisch eine beispielhafte Ausführungsform eines Röntgendetektors 1.

Der erfindungsgemäße Röntgendetektor 1 ist in diesem Beispiel Teil eines Detektormoduls mit mehreren erfindungsgemäßen Röntgendetektoren 1. In einer bevorzugten Ausführungsform weist das Detektormodul eine zweidimensionale Matrix oder Anordnung einer Mehrzahl von Röntgendetektoren 1 auf. Ein jeweiliger Röntgendetektor 1 weist wiederrum eine Vielzahl an Pixelelementen 5 in einer matrixartigen Anordnung auf.

Ein jeweiliger Röntgendetektor 1 in dem gezeigten Beispiel weist ein Konverterelement 3 auf. Das Konverterelement 3 kann als flächenhafter Direktkonverter, beispielsweise aufweisend CdTe, CZT, CdZnTeSe, CdTeSe, CdMnTe, InP, TlBr2, HgI2, GaAs, Si oder andere als Konvertermaterial, ausgebildet sein. Die Oberseite des Konverterelements 3 weist eine erste Elektrode 18 (Top-Elektrode) auf. Die Unterseite des Konverterelements 3 weist Sensor-Pixelelektroden 16 auf. Die Sensor-Pixelelektroden 16 sind über die elektrisch leitende Verbindungen 69 und die Pixelelektroden 57 mit der Auswerteeinheit 59 verbunden. Die Auswerteeinheit 59 kann beispielsweise einen ASICS ("Application Specific Integrated Circuit", dt.: Anwendungsspezifische integrierte Schaltung), umfassen. Die elektrisch leitenden Verbindungen 69 können beispielsweise als Lotkugeln ("bump bonds") oder Lotmaterial in Verbindung mit Kupfersäulen ("copper pillars") oder auch anderweitig ausgebildet sein. Die gemeinsame Anzahl der Sensor-Pixelelektroden 16, die Anzahl der leitenden Verbindungen 69, die Anzahl der Pixelelektroden 57 und die Anzahl der Pixelelemente 5 sind in der Regel gleich. Ein elektrisches Feld zwischen der ersten Elektrode 18 und einer jeweiligen Sensor-Pixelelektrode 16 bestimmt ein einem Pixelelement 5 der Vielzahl an Pixelelementen 5 jeweils zugeordnetes sensitives Detektionsvolumen im Konverterelement 3.

In dem gezeigten Beispiel ist zwischen dem Konverterelement 3 und der Auswerteeinheit 59 eines jeweiligen Röntgendetektors 1 gemäß einer vorteilhaften Ausgestaltung außerdem eine Umverdrahtungslage 65, auch Interposer genannt, angeordnet. Eine Umverdrahtungslage 65 umfasst insbesondere elektrisch leitende Verbindungen, welche eine erste räumliche Verteilung von Eingangskontaktelementen auf eine zweite räumliche Verteilung von Ausgangskontaktelementen überträgt. Die Eingangskontaktelemente der Umverdrahtungsschicht 65 sind dann insbesondere auf einer den Sensor-Pixelelektroden 16 zugewandten Seite der Umverdrahtungslage 65 angeordnet und mit diesen signaltechnisch gekoppelt. Die Ausgangskontaktelemente sind dann insbesondere auf einer den Pixelelektroden 57 der Auswerteeinheit 59 zugewandten Seite der Umverdrahtungslage 65 angeordnet und signaltechnisch mit diesen gekoppelt. Die räumliche Verteilung der Eingangskontaktelemente entspricht dabei im Wesentlichen der räumlichen Verteilung der Sensor-Pixelelektroden 16. Die räumliche Verteilung der Ausgangskontaktelemente entspricht dann im Wesentlichen den Pixelelektroden 57 der Auswerteeinheit 59. Die Umverdrahtungslage 65 kann auch in direktem Kontakt und auf dem Konverterelement 3 oder in direktem Kontakt und auf der Auswerteeinheit 59 aufgebracht sein.

Die Umverdrahtungslage 65 erstreckt sich in dem gezeigten Beispiel dabei über eine Mehrzahl an Auswerteeinheiten 59 und Konverterelementen 3. Dies kann insbesondere vorteilhaft für die Stabilität des Röntgendetektors sein. Es kann jedoch auch andere Ausführungen geben. Ebenso kann es Ausführungsvarianten geben, bei denen mehreren Auswerteinheiten 59 einem gemeinsamen flächig ausgebildeten Konverterelement 3 zugeordnet sind.

Darüber hinaus kann der Röntgendetektor 1 oder das Röntgendetektormodul auch noch weitere, hier nicht gezeigte Komponenten, beispielsweise ein Substrat oder periphere Elektronik, umfassen.

Die Auswerteeinheit 59 ist ausgebildet elektrische Signale, hervorgerufen durch auf das Konverterelement 3 eintreffende Röntgenstrahlung, zu verarbeiten. Erfindungsgemäß weist dabei jedes Pixelelement 5 der Vielzahl an Pixelelementen 5 des Röntgendetektors 1 eine erste, insbesondere pixelweise, Signalverarbeitungsstufe für die Verarbeitung der elektrischen Signale auf, wobei an einem jeweiligen Signalausgang 7 der ersten Signalverarbeitungsstufe eines jeweiligen Pixelelements 5 der Vielzahl an Pixelelementen ein digitales Pixelsignal bereitgestellt wird.

Erfindungsgemäß sind weiterhin die Signalausgänge 7 der ersten Signalverarbeitungsstufe von zumindest einer Gruppe 20 von Pixelelementen 5 der Vielzahl an Pixelelementen 5 mit einer gemeinsamen zweiten Signalverarbeitungsstufe signaltechnisch gekoppelt. Die zweite Signalverarbeitungsstufe dient dann insbesondere der digitalen Weiterverarbeitung der bereitgestellten digitalen Pixelsignale der Gruppe 20 von Pixelelementen 5.

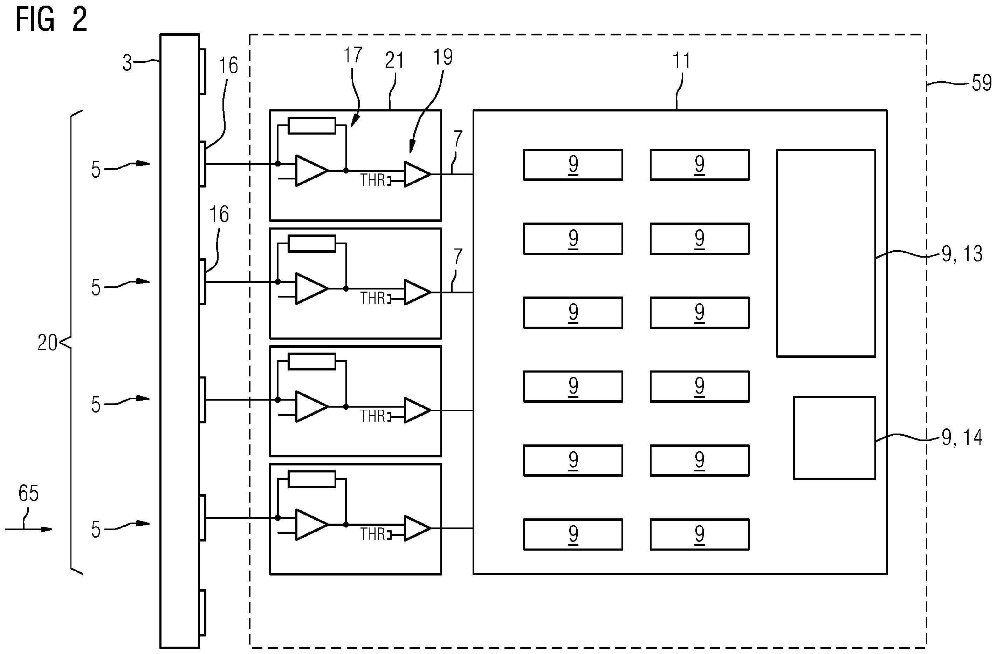

Die Fig. 2 zeigt ein Schema zur Veranschaulichung einer signaltechnischen Verschaltung einer Gruppe 20 an Pixelelementen 5 der Vielzahl an Pixelelementen 5 mit einer ersten und einer zweiten Signalverarbeitungsstufe in einer stark abstrahierten Form.

Die Anzahl der Pixelelemente 5 einer Gruppe 20 ist dabei rein exemplarisch und lediglich zur Veranschaulichung gewählt. Es können auch mehr oder weniger Pixelelemente 5 einer Gruppe 20 zugeordnet sein.

Die einem jeweiligen Pixelelement 5 der Gruppe 20 zugeordneten Sensorpixelelektrode 16 ist mit der in der Auswerteeinheit 59 bereitgestellten ersten Signalverarbeitungsstufe signaltechnisch gekoppelt. Insbesondere weist jedes Pixelelement 7 eine erste pixelweise Signalverarbeitungsstufe auf.

Die erste Signalverarbeitungsstufe eines Pixelelements 5 für die Verarbeitung der vom Konverterelement 3 eingespeisten Signale weist jeweils zumindest einem Signalverstärker 17 und zumindest einem Komparator 19 auf. Der Komparator weist zumindest einen einstellbaren Schwellwert THR auf.

Ein über die Pixelelektrode eingegangene Signal wird in der ersten Signalverarbeitungsstufe eines Pixelelements 5 mittels des Signalverstärkers 17 verstärkt und das verstärkte Signal mittels des zumindest einen Komparator 19 mit zumindest dem Schwellwert THR, repräsentierend eine Energieschwelle, verglichen. In dem Falle, dass das Signal den Schwellwert THR übersteigt, wird ein Ausgangsignal am Signalausgang 7 ausgegeben. Am Signalausgang 7 der ersten Signalverarbeitungsstufe wird insbesondere ein digitales Pixelsignal bereitgestellt.

Die erste Signalverarbeitungsstufe eines Pixelelements 5 der Vielzahl an Pixelelementen 5 kann auch mehr als einen Komparator 19 mit jeweils einem Schwellwert THR aufweisen, so dass mehrere digitale Pixelsignale in Abhängigkeit der mehreren Schwellwerte THR an mehreren Signalausgängen 7 eines Pixelelements 5 der Vielzahl an Pixelelementen bereitstellbar sind.

Die Signalausgänge 7 der ersten Signalverarbeitungsstufe zumindest der Gruppe 20 von Pixelelementen 5 der Vielzahl an Pixelelementen 5 sind weiter mit einer gemeinsamen zweiten Signalverarbeitungsstufe signaltechnisch gekoppelt.

Die gemeinsame zweite Signalverarbeitungsstufe weist eine Mehrzahl digitaler Logikelemente 9 zur digitalen Verarbeitung der an den Signalausgängen 7 bereitgestellten, digitalen Pixelsignale auf. Die in der zweiten Signalverarbeitungsstufe bereitgestellte Mehrzahl an digitalen Logikelementen 9 können als bereitgestellte gemeinsame Ressourcenelemente für die digitale Signalverarbeitung der digitalen Pixelsignale verstanden werden, welche für die Signalverarbeitung der digitalen Pixelsignale der Pixelelemente 5 der Gruppe der Vielzahl an Pixelelementen gemeinsam nutzbar sind.

Außerdem umfasst die gemeinsame zweite Signalverarbeitungsstufe eine konfigurierbare Schaltmatrix 11 zur signaltechnischen Verschaltung zumindest einer Teilzahl der Mehrzahl an digitalen Logikelementen 9 mit den jeweiligen Signalausgängen 7 der ersten Signalverarbeitungsstufe der Gruppe 16 von Pixelelementen 5 der Vielzahl an Pixelelementen.

Nach einer Konfiguration der Schaltmatrix 11 kann dann für jeden Signalausgang 7 der ersten Signalverarbeitungsstufe der Gruppe 16 von Pixelelementen 5 der Vielzahl an Pixelelementen eine Verarbeitungskette für die digitale Verarbeitung der bereitgestellten, digitalen Pixelsignale bereitgestellt werden. Die Verarbeitungskette kann also insbesondere die Ver- und Hintereinanderschaltung einer Auswahl an digitalen Logikelemente 9 und eines jeweiligen Signalausgangs 7 darstellen, so dass durch die Verschaltung die digitale Verarbeitung eines an dem Signalausgang 7 ausgegebenen digitalen Pixelsignals vergebenen ist.

Die Schaltmatrix 11 kann dabei ähnlich wie ein FPGA ("fieldprogrammable gate array", übersetzbar in "im Feld programmierbare (Logik-)Gatter-Anordnung") ausgebildet sein. Das heißt die Schaltmatrix 11 kann als integrierter Schaltkreis, in welchen eine logische Schaltung geladen werden kann, ausgebildet sein, wobei durch eine Programmierung bzw. Konfiguration der Schaltmatrix 11 (nicht nur eine Vorgabe zeitlicher Abläufe, sondern auch) die gewünschte Schaltungsstruktur definierbar, d.h. anpassbar, ist.

Die Auswahl an digitalen Logikelementen 9 umfasst dabei zumindest eine Teilzahl der bereitgestellten digitalen Logikelemente 9. Die Logikelemente können jeweils mit einem oder mehreren Signalausgängen 7 der ersten Signalverarbeitungsstufe und/oder jeweils wieder mit einem oder mehreren digitalen Logikelementen 9 verschaltet sein.

Dabei kann, nach einer Konfiguration, für eine erste Anzahl der Signalausgänge 7 der Gruppe 16 von Pixelelementen 5 eine erste Verarbeitungskette und für eine zweite Anzahl an Signalausgängen 7 eine von der ersten Verarbeitungskette verschiedene zweite Verarbeitungskette bereitgestellt sein.

Gemäß einer weiteren vorteilhaften Ausführungsvariante ist die konfigurierbare Schaltmatrix 11 außerdem insbesondere mittels einstellbarer Registerparametern konfigurierbar, so dass in Abhängigkeit der eingestellten Registerparameter unterschiedliche digitale Verarbeitungsketten für die Signalausgänge 7 bereitstellbar sind.

Die Mehrzahl an digitalen Logikelemente 9 umfasst zumindest ein Zählelement 13 und ein Ausleseelement 14.

Dadurch kann in einer einfachsten Konfiguration des Röntgendetektors 1 ein einfacher photonenzählender Röntgendetektor bereitgestellt werden, welcher die Anzahl direkt in einem Pixelelement 5 eingegangener Signale in Abhängigkeit der bereitgestellten Schwellwerten THR eines jeweiligen Pixelelements 5 zählt, worauf basierend ein Röntgenbilddatensatz erstellt werden kann.

Ein Zählelement 13 umfasst eine Mehrzahl an Zählern.

Ein Zähler kann insbesondere dazu ausgebildet sein, bei Eingang eines Signals einen Zählerstand des Zählers um eine Zähleinheit zu erhöhen. Der Zähler kann beispielsweise einen sogenannten "rising edge counter" oder "falling edge counter" (Zähler basierend auf einer ansteigenden Flanke oder basierend auf einer fallenden Flanke eines Signals) umfassen. Das Zählelement 13 kann also einem Ressourcenblock aufweisend eine Mehrzahl an Zählern entsprechen, so dass beispielsweise basierend auf mehreren mit dem Zählelement 13 gekoppelten Signalausgängen 7 jeweils eine Anzahl an erzeugten digitalen Pixelsignalen gezählt und zumindest zeitweise gespeichert werden kann.

Mittels eines Ausleseelements 14 können dann die Zählerstände des Zählelements 14 ausgelesen werden. Ein Ausleseelement 14 kann beispielsweise ein Schieberegister oder einen sogenannten Readout-Tree ("Auslese-Baum") umfassen.

Darüber hinaus kann die Mehrzahl an digitalen Logikelementen 9 gemäß einer vorteilhaft zweckmäßigen Ausführungsvariante außerdem zumindest ein Element der folgenden Liste umfassen: eine Koinzidenzlogik, ein Signalverzögerungselement, ein Pufferelement, ein Schaltelement zur Verhinderung einer Paralyse eines Zählelements, ein Kombinatorisches Logikgatter, ein Multiplexer, ein Registerelement, ein Element, welches bei Auslösen einen Puls fester oder konfigurierbarer Länge erzeugt, ein Element, welches den Zeitpunkt oder die Zeitdauer eines Triggers misst.

Durch das Bereitstellen zumindest eines oder mehrerer der genannten Elemente können in vorteilhafter Weise zweckmäßige Verschaltungen für den Einsatz des photonenzählenden Röntgendetektors realisiert werden.

Eine Koinzidenzlogik ermöglicht beispielsweise Koinzidenzen zwischen zwei oder mehr Pixelelementen 5 der Vielzahl an Pixelelementen zu registrieren. Die Sammlung von Koinzidenzinformation und das Eingehen der Koinzidenzinformation in den Röntgenbilddatensatz kann eine verbesserte Ortsauflösung und/oder spektrale Auflösung ermöglichen.

Ein Signalverzögerungselement kann insbesondere vorteilhaft eingesetzt werden um Signalunterschiede zwischen zwei digitalen Pixelsignalen, beispielsweise durch unterschiedliche Leitungslängen, auszugleichen.

Ein Schaltelement zur Verhinderung einer Paralyse kann vorteilhaft eingesetzt werden, um ein Hochflussverhalten des Röntgendetektors zu verbessern. Ein solches Schaltelement kann bei dauerhafter Überschreitung einer Komparatorschwelle weitere Signale induzieren. Ein solches Schaltelement kann beispielsweise als Pile-Up Trigger (siehe beispielsweise Kraft et al. "Experimental evaluation of the pile-up trigger method in a revised quantum-counting CT detector", Proc. SPIE 8313, Medical Imaging 2012: Physics of Medical Imaging, 83134A (2012); https://doi.org/10.1117/12.911231) oder als sogenannter "instant retrigger" (Loeliger et al. "The new PILATUS3 ASIC with instant retrigger capability", 2012 IEEE Nuclear Science Symposium and Medical Imaging Conference Record (NSS/MIC) (2012); https://doi.org/610-615.10.1109/NSSMIC.2012.6551180) ausgebildet sein.

Ein kombinatorisches Logikgatter kann ein AND-, NAND-, OR-, NOR-, XOR- oder NOT-Gattern umfassen. Vorteilhaft können darauf basierend verschiedene arithmetische bzw. logische Funktionen umgesetzt werden, beispielsweise ein Addieren oder ein Subtrahieren von Signalen. Außerdem können Elemente zur Datenübertragung, beispielsweise ein Multiplexer, Demultiplexer, Encoder, Decoder umgesetzt werden.

Ein Multiplexer kann beispielsweise vorteilhaft aus einer Anzahl von Eingangssignalen eines ausgewählt an den Ausgang des Multiplexers durchschalten.

Mittels eines Elements, welches bei Auslösen einen Puls fester oder konfigurierbarer Länge erzeugt, kann beispielsweise eine, im Fachgebiet photonenzählender Detektoren breit bekannte, sogenannte "Time-over-Threshold"-Messung (Dauer einer Schwellüberschreitung) erlauben. Ein Element, welches den Zeitpunkt oder die Zeitdauer eines Triggers misst, kann beispielsweise eine, im Fachgebiet photonenzählender Detektoren breit bekannte, sogenannte "Time-of-Arrival" Messung (Ankunftszeit eines Signals) erlauben. Beispielsweise kann dies vorteilhaft für die Sammlung von Koinzidenzinformation genutzt werden.

Ein Registerelement kann vorteilhat eine Konfiguration mittels im Registerelement abgelegter Registerparameter, eine Synchronisation, und/oder Auslese ermöglichen.

Ein Pufferelement kann vorteilhaft zum Treiben von Knoten mit höheren Lasten vorgesehen sein.

Darüber hinaus können auch andere Arten von Logikelemente vorgesehen sein. Beispielsweise kann ein Logikelement vorgesehen sein, das bei Auftreten eines (bestimmten oder konfigurierbaren) Musters auf seinen Eingängen auslöst. Ebenso können Verbindungselemente zu einem oder mehreren Taktsignalen vorgesehen sein oder Monitoring-Elemente (z.B. Digitale Buffer für die externe Beobachtung eines Signals) vorgesehen sein.

Bevorzugt kann insbesondere ein jeweilige Logikelement 9 der Mehrzahl an Logikelemente 9 in der zweiten Signalverarbeitungsstufe auch mehrfach bereitgestellt sein. Die jeweiligen digitalen Logikelemente 9 kann es dann je nach Einsatzzweck und nach verfügbarem Platz und verfügbarer Leistung in geeigneter Auswahl und Anzahl für eine zweckmäßige Bereitstellung von Signalverarbeitungsketten für eine Mehrzahl an Signalausgängen 7 geben.

Vorteilhaft kann bei einer Planung und der Synthese der Digitallogik außerdem durch geeignete Platzierung oder geeignete Syntheserandbedingungen sichergestellt werden, dass die typischen Verbindungswege und Knotenimpedanzen vor allem im schnell schaltenden Bereich optimiert werden. Beispielsweise kann es sinnvoll sein, die Koinzidenzlogiken verteilt jeweils zwischen benachbarten Pixelelementen 5 zu platziert.

Es kann vorteilhafterweise für einen ressourcenschonenden und verbrauchseffizienten Röntgendetektor 1 vorgesehen sein, dass nach einer Konfiguration der konfigurierbaren Schaltmatrix 11 nicht verschaltete Logikelemente 9 der Mehrzahl an Logikelementen 9 von einer Leistungsversorgung getrennt sind.

Gemäß einer weiteren Ausgestaltungsvariante des photonenzählenden Röntgendetektors 1 ist die Vielzahl an Pixelelemente 5 in eine Mehrzahl an Gruppen 20 von Pixelelementen 5 unterteilt, wobei jeder Gruppe 20 jeweils eine konfigurierbare Schaltmatrix 11 zugeordnet ist. Eine Gruppe kann beispielsweise jeweils einem ASIC zugeordnet sein. Eine Gruppe kann auch eine anderweitige Auswahl an Pixelelementen 5 umfassen, beispielsweise jeweils ein ASG-Pixel oder ein Makro-Pixel.

Zwischen den Gruppen 20, d.h. also auch zwischen den Schaltmatrizen können dennoch signaltechnische Verbindungen vorgesehen sein, so dass auch ein Signalaustausch über Gruppen hinweg, beispielsweise für eine Koinzidenz-Betrachtung möglich ist.

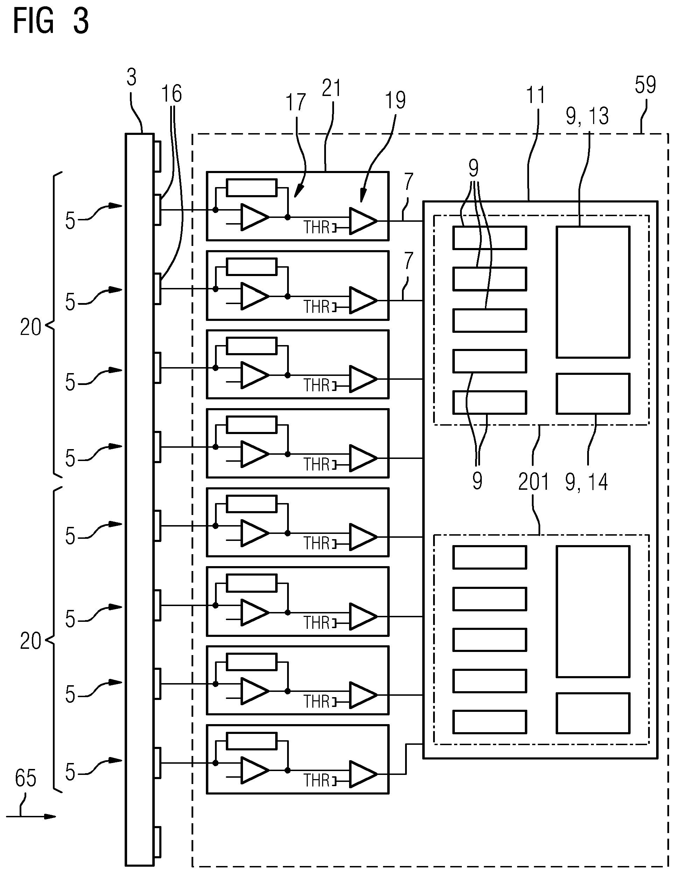

Wie in Fig. 3 schematisch angedeutet, können auch mehrere Gruppen 20 an Pixelelementen 5 einer Schaltmatrix 11 zugeordnet sein. Das heißt, die Vielzahl an Pixelelemente 5 ist in eine Mehrzahl an Gruppen 20 von Pixelelementen 5 unterteilt, wobei der Mehrzahl an Gruppen 20 zusammen eine konfigurierbare Schaltmatrix 11 zugeordnet ist.

Dabei kann jedoch bevorzugt vorgesehen sein, dass die eine konfigurierbare Schaltmatrix 11 gruppenbasiert konfigurierbar ist, so dass die Konfiguration einer Teilmatrix der Schaltmatrix auf den Rest der Schaltmatrix gruppenbasiert repliziert werden kann.

Dabei kann vorgesehen sein, dass die gleiche Anzahl und Auswahl an digitalen Logikelementen (angedeutet durch die Einrahmung 201 in Fig. 3) wiederholt für jede Gruppe vorgesehen ist, so dass für jede Gruppe 20 an Pixelelementen die gleichen Signalverarbeitungsketten für die digitalen Pixelsignale mittels der Schaltmatrix 11 konfigurierbar sind.

Durch die Konfigurierbarkeit der Schaltmatrix 11 und damit der Signalverarbeitungsketten ergeben sich eine Vielzahl an Möglichkeiten für die Signalverarbeitung. So kann die Auswahl und Verschaltung der Logikelemente je nach Röntgenanwendung angepasst werden: Bei sehr niedrigen Röntgenflüssen, z.B. im Rahmen von Screeninganwendungen, kann beispielsweise eine Konfiguration mit einem gewichteten Pixelfusing (Vereinigung der digitalen Pixelsignale mehrerer Pixelelemente) oder mit maximaler räumlicher und/oder spektraler Auflösung vorteilhaft sein. Bei einer Hochflussanwendung hingegen kann beispielsweise die Nutzung von Koinzidenzlogiken zur Reduktion oder zur Messung des negativen Einflusses von Charge-Sharing/Pulse-Pile-Up aktiviert werden. Bei Untersuchungen, die eine hohe Ortsauflösung erfordern (z.B. bei Untersuchungen des Gehörs oder bei Osteoporose) kann eine Konfiguration vorteilhaft sein, welche die Zählerressourcen zur Maximierung der Ortsauflösung einsetzt und dafür die Signale in Abhängigkeit einer geringeren Zahl an spektralen Schwellen vermisst. Bei Untersuchungen, die eher einen hohen Weichteilkontrast oder eine präzise Materialidentifikation benötigen, kann eine gegenteilige Konfiguration (Rauschreduktion durch geringere Ortsauflösung, gewichtetes Pixelfusing, hohe Zahl spektraler Schwellen bzw. spektrale Gewichtung) vorteilhaft sein. Bei zeitkritischen Untersuchungen (z.B. in der Kardiologie oder in der Notaufnahme) kann hingegen eine Messung mit maximaler Bildfrequenz wünschenswert sein, um eine Minimierung von Scandauer und Bewegungsartefakten erreichen. Dies kann durch eine Konfiguration mit minimalem Datenvolumen erreicht werden. Durch Reduktion der spektralen Auflösung und räumlichen Auflösung (durch Kombination von Nachbarpixeln) lässt sich beispielsweise das Rohdatenvolumen senken. Dies kann beispielsweise mittels Summation der Komparatorsignale benachbarter Pixel (Veroderung) oder durch die Addition benachbarter Zählerstände direkt auf dem Chip erreicht werden. Dies hat neben der Ermöglichung kürzerer Ausleseintervalle insbesondere den Vorteil, dass gleichzeitig auch die Leistungsaufnahme während der Auslese sinkt. Bei Untersuchungen mit unklarer oder polymorbider Fragestellung (Patienten mit Mehrfacherkrankung) hingegen kann eine Konfiguration sinnvoll sein, die weniger Geschwindigkeit bietet, dafür aber die maximale Informationsfülle in einem einzelnen Scandurchgang liefert.

Ferner kann auch die räumliche und spektrale Auflösung unabhängig voneinander konfigurierbar sein. Ein konkretes Beispiel kann etwa umfassen eine erste und zweite Schwelle in voller Ortsauflösung zu messen, jedoch eine dritte Schwelle lediglich mit halber Ortsauflösung zu messen, dafür jedoch die freien Zählerressourcen für einen Koinzidenzzähler in Verbindung mit der ersten Schwelle vorzusehen.

Vorteilhafterweise kann außerdem die Flexibilität u. Leistungsfähigkeit der Koinzidenzschaltungen verbessert bereitgestellt werden. Durch die flexible Verschaltung können je nach Anwendung beispielsweise auch Koinzidenzen höherer Ordnung ermittelt werden, beispielsweise eine Koinzidenz eines zweiten, höherenergetischen Schwellwerts THR in einem Pixelelement mit einem Signal mit einem ersten, niederenergetischeren Schwellwert eines Nachbarpixels.

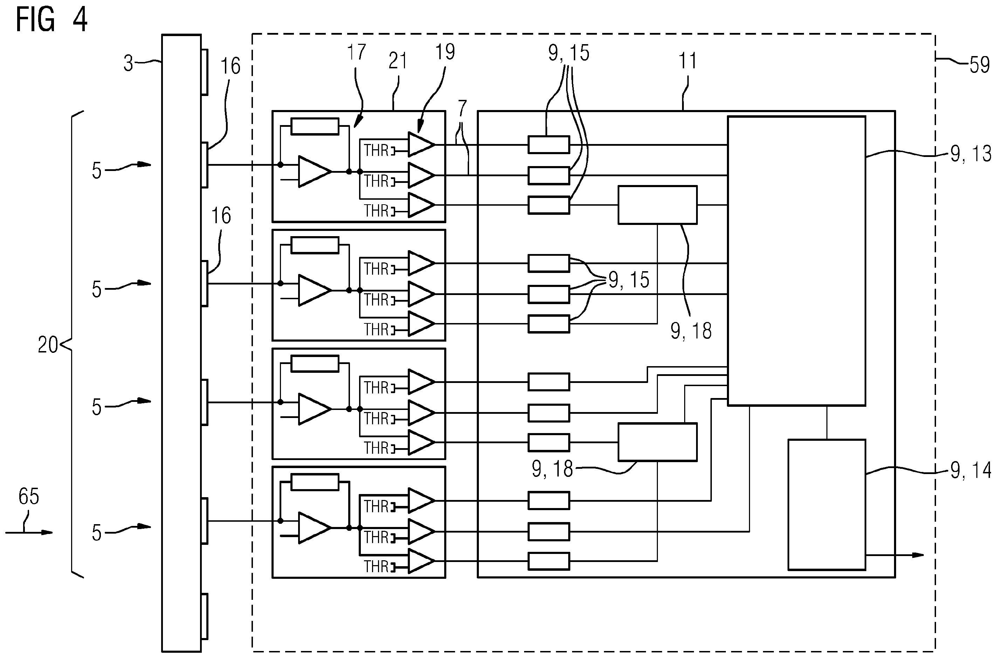

Die Fig. 4 zeigt ein Schema zur Veranschaulichung einer signaltechnischen Verschaltung einer Gruppe 20 an Pixelelementen 5, wobei eine beispielhafte Verarbeitungskette gemäß einer ersten Konfigurationsvariante veranschaulicht ist.

Insbesondere sind in der Darstellung nur diejenigen digitalen Logikelemente 9 durch Kästen angedeutet, welche in dieser vereinfachten Variante verschaltet sind. Darüber hinaus können weitere, hier nicht gezeigte, Logikelemente 9 in der zweiten Signalverarbeitungsstufe bereitgestellt sein.

In dieser Variante weist die erste pixelweise Signalverarbeitungsstufe eines jeweiligen Pixelelements 5 drei Komparatoren 19 und jeweils drei Signalausgänge 7 auf. Jeder Signalausgang 7 der Pixelelemente der Gruppe 20 ist mit der gemeinsamen zweiten Signalverarbeitungsstufe aufweisend die konfigurierbare Schaltmatrix 11 und die Mehrzahl an Logikelementen 9 gekoppelt.

Die Signalausgänge 7 des jeweiligen ersten und zweiten Komparators 19 jedes Pixelelements 5 ist mit dem Zählelement 13 aufweisend eine Mehrzahl an Zählern verschaltet, so dass basierend auf den Ausgangssignalen der jeweiligen ersten und zweiten Komparatoren 19 in Abhängigkeit der Schwellwerte THR jeweils eine Anzahl von digitalen Pixelsignalen gezählt werden kann.

Darüber hinaus ist der Signalausgang 7 des jeweils dritten Komparators 19 eines Pixelelements 5 gemeinsam mit dem entsprechenden Signalausgang 7 eines benachbarten Pixelelements 5 über ein Summationselement 18 mit dem Zählelement 13 verschaltet, so dass in Abhängigkeit des Schwellwertes THR des jeweils dritten Komparators 19 eine summierte Anzahl an Pixelsignalen jeweils zweier benachbarter Pixelelemente 5 gezählt wird.

Daraus resultiert, dass in dieser Konfigurationsvariante basierend auf der ersten und der zweiten Komparatorschwelle THR digitale Pixelsignale in voller Ortsauflösung gezählt werden, wohingegen die dritte Komparatorschwelle THR lediglich mit einer halben Ortsauflösung vermessen wird. Dabei wird angenommen, dass die Schwellwerte der Pixelsignalen gleich eingestellt sind. Das heißt, der jeweils erste Komparator 19 weist jeweils die gleiche Energieschwelle über alle Pixelelemente 5 auf, ebenso wie der jeweils zweite Komparator 19 und der jeweils dritte Komparator 19.

Darüber hinaus ist jeder Signalausgang 7 in diesem Beispiel mit einem Element zur Verhinderung einer Paraylse des jeweils gekoppelten Zählers für ein verbessertes Hochflussverhalten verschaltet.

Die Fig. 4 zeigt ein Schema zur Veranschaulichung einer signaltechnischen Verschaltung einer Gruppe 20 an Pixelelementen 5, wobei eine beispielhafte Verarbeitungskette gemäß einer zweiten Konfigurationsvariante dargestellt ist.

In dieser Variante weist die erste pixelweise Signalverarbeitungsstufe eines jeweiligen Pixelelements 5 erneut drei Komparatoren 19 und jeweils drei Signalausgänge 7 auf. Jedoch werden jeweils nur die digitalen Pixelsignale, welche basierend auf den Signalausgängen 7 der ersten zwei Komparatoren 19 bereitgestellt werden, weiterverarbeitet. Dafür sind jeweils die Signalausgänge 7 basierend auf dem jeweils ersten Komparator 19 von jeweils zwei benachbarten Pixelelementen 5 zusätzlich mit einer Koinzidenzlogik 12 gekoppelt.

In dieser Variante werden folglich erneut Signale basierend auf den ersten zwei Schwellwerten eines jeden Pixelelements 5 mittels des Zählelements 13 gezählt und darüber hinaus koinzidierend auftretende Signale zwischen jeweils zwei benachbarten Pixelelementen 5. In diesem Beispiel wird auf die spektrale Auflösung verzichtet, um im Gegenzug die Koinzidenzinformation zwischen jeweils zwei benachbarten Pixelelementen 5 zu sammeln.

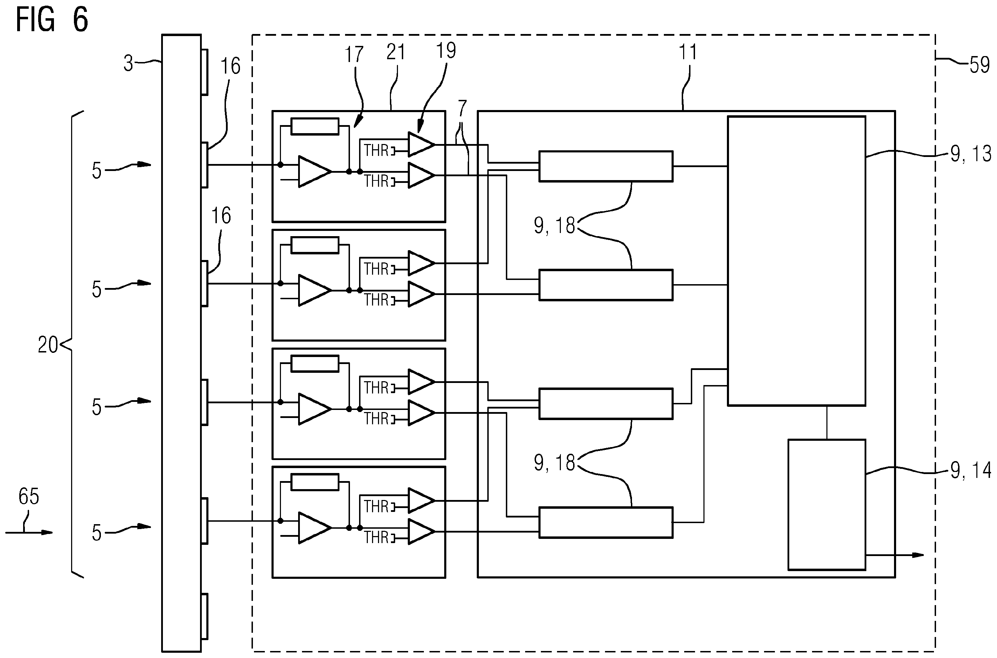

Fig. 6 zeigt ein Schema zur Veranschaulichung einer signaltechnischen Verschaltung einer Gruppe 20 an Pixelelementen 5, wobei eine beispielhafte Verarbeitungskette gemäß einer dritten Konfigurationsvariante dargestellt ist.

In diesem Fall werden die digitalen Pixelsignale sich entsprechender Komparatoren 19 jeweils zweier benachbarter Pixelelemente 5 über eine Summationslogik 18 zusammengeführt und mittels des Zählelements 13 gezählt, womit zwar eine geringere Ortsauflösung aber ein Reduktion des Datenvolumens und damit eine schnelle Aufnahmefrequenz erreicht werden kann.

Die oben beschriebenen Konfigurationsvarianten stellen dabei einfache Verschaltungen zur exemplarischen Veranschaulichung dar. Es sind darüber hinaus wesentlich komplexere Konfigurationsvarianten umsetzbar und in einfacher Weise aus den beschriebenen Varianten ableitbar.

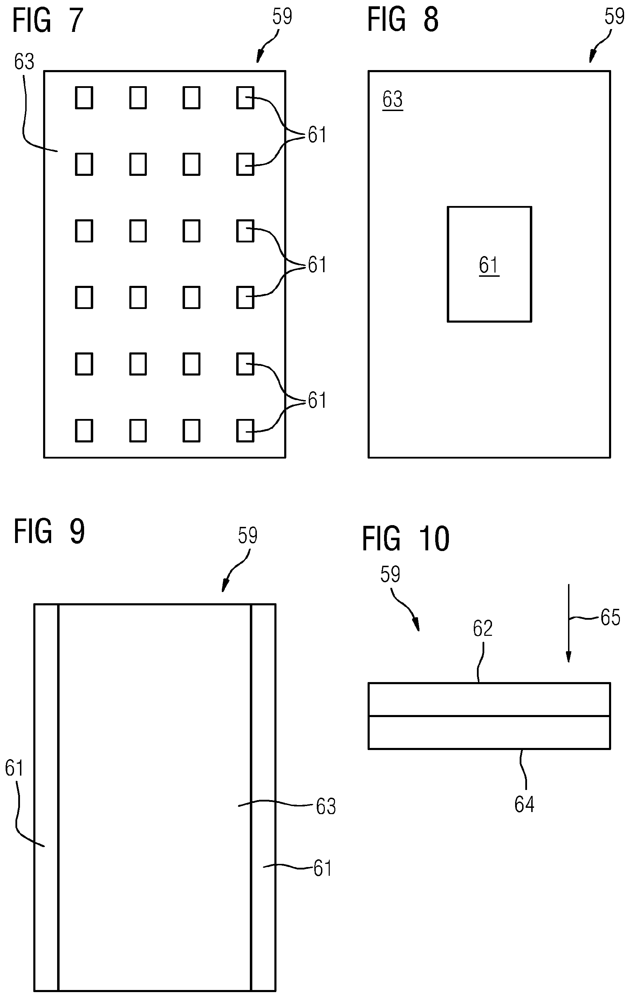

Fig. 7 bis 9 zeigen jeweils eine Veranschaulichung einer beispielhaften relativen Anordnung der für eine erste und eine zweite Verarbeitungsstufe vorgesehenen Bereiche einer Auswerteeinheit 59, welche die erste Verarbeitungsstufe und die zweite Verarbeitungsstufe für zumindest die eine Gruppe 20 an Pixelelementen 5 der Vielzahl an Pixelelementen bereitstellt.

Der Bereich kann als Flächenbereich der Auswerteeinheit 59 verstanden werden. Ein jeweiliger Flächenbereich 59 kann insbesondere flächenhaft senkrecht zu einer Strahleneinfallsrichtung bzw. Stapelrichtung der Stapelanordnung aus Konverterelement 3 und Auswerteeinheit 59 ausgebildet sein.

Gemäß einer Ausführungsvariante des Röntgendetektors 1 kann die Auswerteeinheit 59 zumindest einen ersten Bereich 61 für die erste Verarbeitungsstufe und einen zweiten Bereich 63 für die zweite Verarbeitungsstufe vorsehen, wobei der zumindest eine erste Bereich 61 inselförmig innerhalb des zweiten Bereich 63, wie beispielhaft in den Figuren 7 und 8 veranschaulicht, vorgesehen ist.

Gemäß einer Ausführungsvariante des Röntgendetektors 1 kann die Auswerteeinheit 59 jedoch auch zumindest einen ersten Bereich 61 für die erste Verarbeitungsstufe und einen zweiten Bereich 63 für die zweite Verarbeitungsstufe vorsehen, wobei der zumindest eine erste Bereich 61 randseitig entlang des zweiten Bereichs 63, wie beispielhaft in Fig. 9 veranschaulicht, vorgesehen ist.

Der für die erste Signalverarbeitungsstufe eines jeweiligen Pixelelements 5 bereitgestellte Bereich 61 der Auswerteeinheit 59 kann jeweils, wie in Fig. 7 dargestellt, inselförmig innerhalb des Bereichs 63, welcher die digitale Signalverarbeitungsstufe für eine Gruppe 20 an Pixelelementen bereitstellt, eingebettet sein. Dabei kann der Bereich 63 der digitalen Signalverarbeitungsstufe beispielsweise die flächenmäßige Ausdehnung der gesamten Pixelgruppe einnehmen.

Ebenso kann vorgesehen sein, die die Bereiche der ersten Signalverarbeitungsstufen einer Gruppe von Pixelelementen auch zu Streifen, Zentren oder Ringen zusammengefasst angeordnet sind. Beispielsweise können die ersten Signalverarbeitungsstufen zusammengefasst mittig und inselförmig wie in Fig. 8 veranschaulicht angeordnet sein. Beispielsweise können die ersten Signalverarbeitungsstufen zusammengefasst jeweils randseitig entlang des zweiten Bereichs 63, wie in Fig. 9 veranschaulicht, angeordnet sein.

Die Fig. 10 zeigt eine Veranschaulichung einer weiteren beispielhaften relativen Anordnung der ersten und eine zweiten Verarbeitungsstufe, wobei die Auswerteeinheit 59 schichtweise aufgebaut ist, und wobei in einer ersten Schicht 62 die erste Verarbeitungsstufe bereitgestellt ist und in einer in Strahleneinfallsrichtung 65 hinter der ersten Schicht 62 gelegenen, zweiten Schicht 64 die zweite Verarbeitungsstufe bereitgestellt ist.

In einer Weiterbildung davon kann eine Aufteilung der analogen und digitalen Schaltkreise, d.h. der jeweiligen ersten Verarbeitungsstufen und der zweiten Verarbeitungsstufe, in separate Wafer-Dies (separate Teilbereiche eines oder verschiedener Halbleiter-Wafer) vorgesehen sein, welche anschließend zu einer Auswerteeinheit 59 zusammengesetzt werden können.

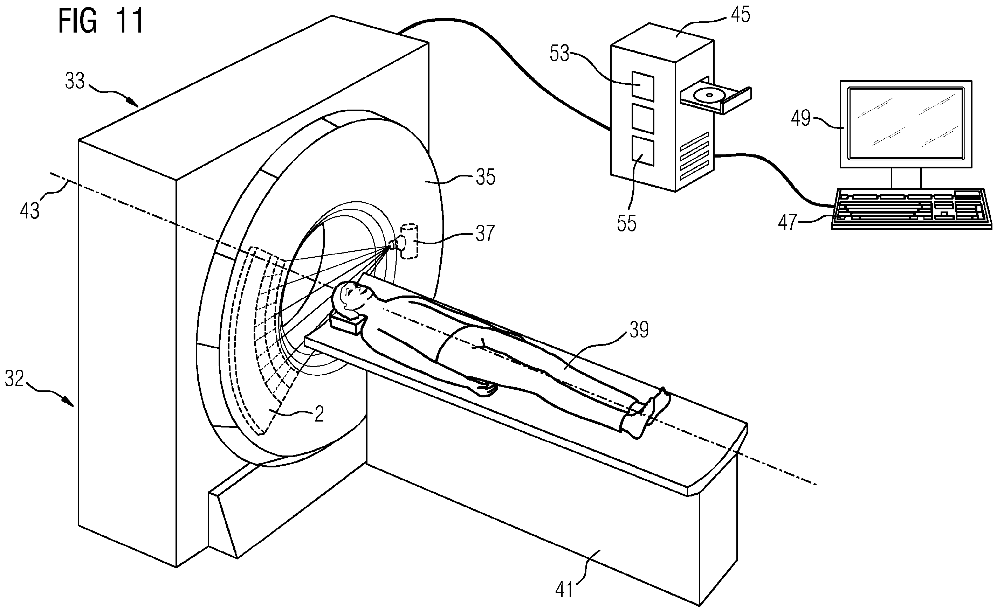

Fig. 11 zeigt eine beispielhafte Ausführungsform eines erfindungsgemäßen medizinischen Bildgebungsgeräts in Form eines Computertomographie-Systems 32. Das Computertomographie-System 32 weist eine Gantry 33 mit einem Rotor 35 auf. Der Rotor 35 umfasst eine Detektorvorrichtung 2 und eine Strahlungsquelle 37, beispielsweise eine Röntgenröhre, zum Belichten der Detektorvorrichtung 2. Die Detektorvorrichtung 2 weist mindestens einen erfindungsgemäßen photonenzählenden Röntgendetektor 1 auf. Die Detektorvorrichtung 2 kann ein Detektormodul mit einer Anzahl an Röntgendetektoren 1 aufweisen.

Das Objekt 39, hier der Patient, ist auf der Patientenliege 41 gelagert und ist entlang der Rotationsachse z 43 durch die Gantry 33 bewegbar. Im Allgemeinen kann das Objekt 39 beispielsweise einen tierischen Patienten und/oder einen menschlichen Patienten umfassen.

Im Falle eines Computertomographie-Systems 32 wird üblicherweise aus einer Vielzahl an Winkelrichtungen jeweils ein (Roh-) Röntgenbilddatensatz des Objekts mittels des Röntgendetektors aufgenommen. Der (Roh-) Röntgenbilddatensatz basiert dann im Wesentlichen auf den ausgegebenen verarbeiteten digitalen Pixelsignalen. Anschließend kann basierend auf den (Roh-)Röntgenbilddatensätzen mittels eines mathematischen Verfahrens, beispielsweise umfassend eine gefilterte Rückprojektion oder ein iteratives Rekonstruktionsverfahren, ein finaler Röntgenbilddatensatz, ein Volumenbilddatensatz oder ein Schichtbilddatensatz, rekonstruiert werden.

Zur Steuerung des medizinischen Bildgebungsgeräts und/oder zur Erstellung eines Röntgenbilddatensatzes basierend auf den mittels des photonenzählenden Röntgendetektors 1 aufgenommenen Messdaten, d.h. den verarbeiteten digitalen Pixelsignalen, ist eine Systemsteuerung in Form einer Recheneinheit 45 vorgesehen.

Die Recheneinheit 45 kann eine Steuereinheit 53 umfassen, welche ausgebildet sein kann, Registerparameterwerte von Registerparametern für eine Konfiguration einer konfigurierbaren Schaltmatrix des photonenzählenden Röntgendetektors 1 anzupassen. Die Steuereinheit 53 kann dazu über eine Steuerschnittstelle mit dem zumindest einen Röntgendetektor gekoppelt sein, um Steuerbefehle und/oder Registerparameterwerte zu übertragen. Die Steuereinheit kann außerdem ausgebildet sein, einen Satz an Registerparameterwerten in Abhängigkeit einer Röntgenanwendung zu bestimmen oder Anwendungsparameter zur Bestimmung eines geeigneten Satzes an Registerparameterwerten abzufragen und/oder zu ermitteln.

Die Steuereinheit 53 und/oder die Recheneinheit 45 kann in Form eines Computers, eines Mikrocontrollers oder eines integrierten Schaltkreises umgesetzt sein. Die Steuereinheit 53 und/oder die Recheneinheit 45 kann Hardware-Elemente oder Software-Elemente aufweisen, beispielsweise einen Mikroprozessor oder ein sogenanntes FPGA (englisches Akronym für "Field Programmable Gate Array"). Es kann sich auch um einen realen oder virtuellen Verbund von Computern handeln (ein englischer Fachbegriff für einen realen Verbund ist "Cluster", ein englischer Fachbegriff für einen virtuellen Verbund ist "Cloud").

Des Weiteren ist eine Eingabeeinrichtung 47 und eine Ausgabeeinrichtung 49 mit der Rechnereinheit 45 verbunden. Die Eingabeeinrichtung 47 und die Ausgabeeinrichtung 49 können beispielsweise eine Interaktion, beispielsweise eine manuelle Anpassung von Registerparameterwerten, die Eingabe eines Parameters basierend auf welchen Registerparameterwerte bestimmt werden können, eine Bestätigung oder ein Auslösen eines Verfahrensschritts durch einen Anwender, ermöglichen. Das medizinische Bildgebungsgerät kann außerdem eine Speichereinheit 55 umfassen.

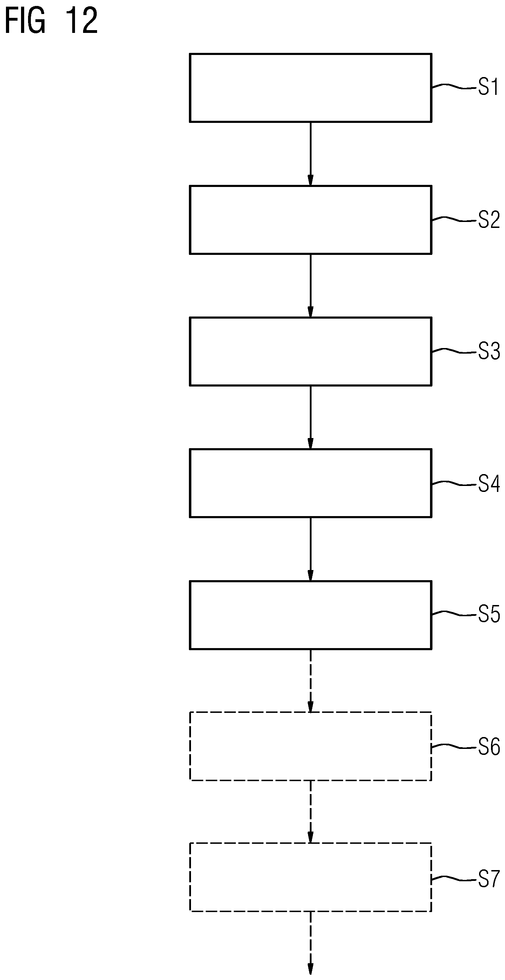

Die Fig. 12 zeigt einen schematischen Verfahrensablauf eines Verfahrens zum Betreiben eines photonenzählenden Röntgendetektors 1, welcher gemäß einer der zuvor beschriebenen Varianten ausgebildet ist, zur Aufnahme eines Röntgenbilddatensatzes, wobei die zumindest eine Schaltmatrix 11 über einstellbare Registerparameter konfigurierbar ist.

Das Verfahren umfasst den Schritt des Anpassens S1 der einstellbaren Registerparameter basierend auf einem ersten Satz an Registerparameterwerten.

Das Verfahren umfasst weiterhin den Schritt S2 Konfigurieren der zumindest einen konfigurierbaren Schaltmatrix 11 basierend auf den angepassten Registerparametern und damit Bereitstellen jeweils einer digitalen Verarbeitungskette für zumindest einen Teil der Signalausgänge 7 der ersten Signalverarbeitungsstufe der zumindest einen Gruppe 20 von Pixelelementen 5, welche mit der zumindest einen Schaltmatrix 11 signaltechnisch gekoppelt sind. Dies umfasst auch, dass für alle Signalausgänge 7 der ersten Signalverarbeitungsstufe der zumindest einen Gruppe 20 von Pixelelementen 5, welche mit der zumindest einen Schaltmatrix 11 signaltechnisch gekoppelt sind jeweils einer digitalen Verarbeitungskette werden kann.

Das Verfahren umfasst weiterhin den Schritt des Empfangens von Röntgenstrahlung mit dem Röntgendetektors 1, worauf basierend elektrische Signale im Konverterelement 3 erzeugt werden, welche im Schritt des Verarbeitens S4 mittels der ersten und der zweiten Verarbeitungsstufe des Röntgendetektors 1 verarbeitet werden, wobei digitale Pixelsignale an einem jeweiligen Signalausgang 7 der ersten Signalverarbeitungsstufe eines Pixelelements 5 der zumindest einen Gruppe 20 von Pixelelementen 5 der Vielzahl an Pixelelementen 5 bereitgestellt werden, welche mittels der für einen jeweiligen Signalausgang 7 bereitgestellten digitalen Verarbeitungskette in der zweiten Signalverarbeitungsstufe weiterverarbeitet werden.

Das Verfahren umfasst weiterhin den Schritt des Ausgebens S5 der verarbeiteten digitalen Pixelsignale, worauf basierend ein Röntgenbilddatensatz ermittelt wird.

Gemäß einer Weiterbildung des Verfahrens einer Ausführungsvariante des erfindungsgemäßen Verfahrens können in einem Schritt S6 des zweiten Anpassens die einstellbaren Registerparameter basierend auf einem zweiten Satz an Registerparametern angepasst werden, und wobei in einem Schritt S7 des zweiten Konfigurierens der zumindest einen konfigurierbaren Schaltmatrix 11 mittels des zweiten Sets an Registerparameterwerten eine zweite, von der ersten verschiedene, digitale Verarbeitungskette für zumindest einen Teil der Signalausgang 7 der ersten Signalverarbeitungsstufe der zumindest einen Gruppe 20 von Pixelelementen 5 der Vielzahl an Pixelelementen 5 bereitgestellt wird.

Gemäß einer vorteilhaften Verfahrensvariante kann dabei der erste und/oder zweite Satz an Registerparametern in Abhängigkeit einer Röntgenanwendung bestimmt werden. Vorteilhaft kann der Röntgendetektor 1 an die momentanen Randbedingungen der Aufnahme des Röntgenbilddatensatzes und der Bedürfnisse der Röntgenanwendung optimal angepasst werden. Dabei ist eine äußerst flexible Anpassung des Röntgendetektors möglich.

Beispielsweise können extern in einer Speichereinheit 55 oder auch in mehreren Registerelementen der Schaltmatrix 11 selbst mehrere Sets an Registerparameterwerten vorgehalten werden. In Abhängigkeit der Röntgenanwendung kann dann ein Satz an Registerparameterwerten aus der Mehrzahl an Sätzen bestimmt und angewendet werden.

Die Sätze an Registerparameterwerten können dabei beispielsweise in Verknüpfung mit einem oder mehreren Anwendungsparametern, welche in Verbindung mit der Röntgenanwendung stehen, vorliegen. Beispielsweise kann ein Anwendungsparameter auf einem Röntgenfluss, einem Zeitpunkt oder einer Zeitdauer, einem Steuerungsparameter des medizinischen Bildgebungsgeräts, beispielsweise ein Röntgenröhrenstrom oder einer Röntgenröhrenspannung, einem Bildparameter des aufzunehmenden Röntgenbilddatensatzes, beispielsweise einer spektralen Auflösung oder einer Ortsauflösung, basieren. Anhand der Anwendungsparameter kann dann ein vorteilhafter Satz an Registerparameterwerten bestimmt werden.

Die Anwendungsparameter können beispielsweise von einer externen Speichereinheit 55, externen Sensoreinheiten oder einer Steuereinheit 53 mittels des Röntgendetektors 1 abgefragt werden. Die Anwendungsparameter können beispielsweise mittels des Röntgendetektors 1 selbst gemessen, d.h. ermittelt werden, beispielsweise ein Röntgenfluss oder eine Energie der Röntgenstrahlung. Basierend auf den abgefragten und/oder ermittelten Anwendungsparametern kann dann ein Satz an Registerparameterwerten bestimmt und für die Konfiguration der Schaltmatrix 11 gewählt werden. Die Anwendungsparameter können auch durch eine mit dem Röntgendetektor 1 gekoppelte Steuereinheit 53 abfragen und/oder ermitteln werden und daraufhin ein Steuerbefehl und/oder neue Registerparameterwerte über eine Steuerschnittstelle an den Röntgendetektor 1 übertragen werden.

Neben Anwendungsparametern, welche in Verbindung mit einer Röntgenanwendung stehen, kann es auch andere Anwendungsparameter geben. Beispielweise kann ein Anwendungsparameter auch auf durch das medizinische Bildgebungsgerät, dem der erfindungsgemäße Röntgendetektor 1 zugeordnet ist, vorgegebenen Randbedingungen basieren, beispielsweise eine zur Verfügung stehende Kühlleistung oder einer Leistungsversorgung.

Vorteilhaft ist eine einfache und gegebenenfalls automatische Konfiguration des Röntgendetektors 1 möglich, so dass stets die für eine Röntgenanwendung, die gegebenen Randbedingungen eines medizinischen Bildgebungsgeräts und/oder Messung günstigste Konfiguration des Röntgendetektors 1 bereitstellbar ist.

## Patentansprüche

1. Photonenzählender Röntgendetektor (1) aufweisend ein Konverterelement (3) zur Umwandlung von Röntgenstrahlung in elektrische Signale und eine Vielzahl an Pixelelementen (5), wobei
a. jedes Pixelelement (5) der Vielzahl an Pixelelementen (5) eine erste Signalverarbeitungsstufe für die Verarbeitung der elektrischen Signale mit jeweils zumindest einem Signalverstärker (17) und zumindest einem Komparator (19) zur Bereitstellung eines digitalen Pixelsignals an einem jeweiligen Signalausgang (7) der ersten Signalverarbeitungsstufe eines Pixelelements (5) der Vielzahl an Pixelelementen (5) aufweist,
b. die Signalausgänge (7) der ersten Signalverarbeitungsstufe von zumindest einer Gruppe (20) von Pixelelementen (5) der Vielzahl an Pixelelementen (5) mit einer gemeinsamen zweiten Signalverarbeitungsstufe signaltechnisch gekoppelt sind, welche eine Mehrzahl digitaler Logikelemente (9) zur digitalen Verarbeitung der bereitgestellten, digitalen Pixelsignale aufweist,
c. die gemeinsame zweite Signalverarbeitungsstufe eine konfigurierbare Schaltmatrix (11) zur signaltechnischen Verschaltung zumindest einer Teilzahl der Mehrzahl an digitalen Logikelementen (9) mit den jeweiligen Signalausgängen (7) der ersten Signalverarbeitungsstufe der Gruppe (20) von Pixelelementen (5) der Vielzahl an Pixelelementen (5) aufweist,
wobei die Mehrzahl an digitalen Logikelementen (9) zumindest ein Zählelement (13) und ein Ausleseelement (14) umfasst,
wobei das zumindest eine Zählelement (13) eine Mehrzahl an Zählern umfasst,
d. so dass nach einer Konfiguration der Schaltmatrix (11) für jeden Signalausgang (7) der ersten Signalverarbeitungsstufe der Gruppe (20) von Pixelelementen (5) der Vielzahl an Pixelelementen (5) eine Verarbeitungskette für die digitale Verarbeitung der bereitgestellten, digitalen Pixelsignale bereitstellbar ist,
und wobei die erste Signalverarbeitungsstufe durch pixelweise Pixelelektroniken ausgebildet ist, so dass einem Pixelelement (5) der Vielzahl an Pixelelementen (5) jeweils eine erste Signalverarbeitungsstufe, eine entsprechende Pixel- und Sensorpixelelektrode (16, 57) und ein Detektionsvolumen im Konverterelement (3) zuordenbar ist, und die zweite Signalverarbeitungsstufe als Ganzes lediglich der Gruppe (20) von Pixelelementen (5) zuordenbar ist.

2. Photonenzählender Röntgendetektor (1) nach Anspruch 1, wobei für eine erste Anzahl der Signalausgänge (7) der Gruppe (20) von Pixelelementen (5) eine erste Verarbeitungskette und wobei für eine zweite Anzahl an Signalausgängen (7) eine von der ersten Verarbeitungskette verschiedene zweite Verarbeitungskette bereitstellbar ist.

3. Photonenzählender Röntgendetektor (1) nach Anspruch 1 oder 2, wobei die Mehrzahl an digitalen Logikelementen (9) außerdem zumindest ein Element der folgenden Liste umfasst
- eine Koinzidenzlogik,
- ein Signalverzögerungselement,
- ein Pufferelement,
- ein Schaltelement zur Verhinderung einer Paralyse eines Zählelements,
- ein Kombinatorisches Logikgatter,
- ein Multiplexer,
- ein Registerelement,
- ein Element, welches bei Auslösen einen Puls fester oder konfigurierbarer Länge erzeugt,
- ein Element, welches den Zeitpunkt oder die Zeitdauer eines Triggers misst.

4. Photonenzählender Röntgendetektor (1) nach einem der Ansprüche 1 bis 3, wobei ein digitales Logikelement (9) der Mehrzahl an digitalen Logikelementen (9) in der zweiten Signalverarbeitungsstufe mehrfach bereitgestellt ist.

5. Photonenzählender Röntgendetektor (1) nach einem der Ansprüche 1 bis 4, wobei nach einer Konfiguration der konfigurierbaren Schaltmatrix (11) nicht verschaltete digitale Logikelemente (9) der Mehrzahl an digitalen Logikelementen (9) von einer Leistungsversorgung getrennt sind.

6. Photonenzählender Röntgendetektor (1) nach einem der Ansprüche 1 bis 5, wobei die Vielzahl an Pixelelementen (5) in eine Mehrzahl an Gruppen (20) von Pixelelementen (5) unterteilt ist, wobei jede Gruppe (20) jeweils einer konfigurierbaren Schaltmatrix (11) zugeordnet ist.

7. Photonenzählender Röntgendetektor (1) nach einem der Ansprüche 1 bis 5, wobei die Vielzahl an Pixelelemente (5) in eine Mehrzahl an Gruppen (20) von Pixelelementen unterteilt ist, wobei der Mehrzahl an Gruppen (20) eine gemeinsame konfigurierbare Schaltmatrix (11) zugeordnet ist.

8. Photonenzählender Röntgendetektor (1) nach Anspruch 7, wobei die eine konfigurierbare Schaltmatrix (11) gruppenweise konfigurierbar ist.

9. Photonenzählender Röntgendetektor (1) nach einem der Ansprüche 1 bis 8, aufweisend eine Auswerteeinheit (59), welche die erste Verarbeitungsstufe und die zweite Verarbeitungsstufe für zumindest eine Gruppe (20) an Pixelelementen (5) bereitstellt, und wobei die Auswerteeinheit (59) zumindest einen ersten Bereich (61) für die erste Verarbeitungsstufe und einen zweiten Bereich (63) für die zweite Verarbeitungsstufe bereitstellt, wobei der zumindest eine erste Bereich (61) inselförmig innerhalb des zweiten Bereichs (63) oder randseitig entlang des zweiten Bereichs (63) angeordnet ist.

10. Photonenzählender Röntgendetektor (1) nach einem der Ansprüche 1 bis 8, aufweisend eine Auswerteeinheit (59), welche die erste Verarbeitungsstufe und die zweite Verarbeitungsstufe für zumindest eine Gruppe (20) an Pixelelementen (5) bereitstellt, und wobei die Auswerteeinheit (59) schichtweise aufgebaut ist, und wobei in einer ersten Schicht (62) die erste Verarbeitungsstufe bereitgestellt ist und in einer in Strahleneinfallsrichtung hinter der ersten Schicht(62) gelegenen, zweiten Schicht (64) die zweite Verarbeitungsstufe bereitgestellt ist.

11. Medizinisches Bildgebungsgerät aufweisend zumindest einen photonenzählenden Röntgendetektor (1) nach einem der voranstehenden Ansprüche.

12. Verfahren zum Betreiben eines photonenzählenden Röntgendetektors (1), welcher gemäß einem der Ansprüche 1 bis 10 ausgeführt ist, zur Erzeugung eines Röntgenbilddatensatzes, wobei die zumindest eine Schaltmatrix (11) des Röntgendetektors (1) mittels einstellbarer Registerparameter konfigurierbar ist, umfassend die Schritte
a. Anpassen (S1) der einstellbaren Registerparameter basierend auf einem ersten Satz an Registerparameterwerten,
b. Konfigurieren (S2) der zumindest einen konfigurierbaren Schaltmatrix (11) basierend auf den angepassten Registerparametern und damit Bereitstellen jeweils einer digitalen Verarbeitungskette für zumindest einen Teil der Signalausgänge (7) der ersten Signalverarbeitungsstufe der zumindest einen Gruppe (20) von Pixelelementen (5), welche mit der zumindest einen Schaltmatrix (11) signaltechnisch gekoppelt ist,
c. Empfangen (S3) von Röntgenstrahlung mit dem photonenzählenden Röntgendetektor (1), worauf basierend elektrische Signale im Konverterelement (3) erzeugt werden,
d. Verarbeiten (S4) der erzeugten elektrischen Signale mittels der ersten Verarbeitungsstufe und der zweiten Verarbeitungsstufe des photonenzählenden Röntgendetektors (1), wobei digitale Pixelsignale an einem jeweiligen Signalausgang (7) der ersten Signalverarbeitungsstufe eines Pixelelements (5) der zumindest einen Gruppe (20) von Pixelelementen (5) der Vielzahl an Pixelelementen (5) bereitgestellt werden, welche mittels der für einen jeweiligen Signalausgang (7) bereitgestellten digitalen Verarbeitungskette in der zweiten Signalverarbeitungsstufe weiterverarbeitet werden,
e. Ausgeben (S5) der verarbeiteten digitalen Pixelsignale, worauf basierend ein Röntgenbilddatensatz erzeugt wird.

13. Verfahren nach Anspruch 12, wobei in einem Schritt (S6) des zweiten Anpassens die einstellbaren Registerparameter basierend auf einem zweiten Satz an Registerparameterwerten angepasst werden und wobei in einem Schritt (S7) des zweiten Konfigurierens der zumindest einen konfigurierbaren Schaltmatrix (11) mittels des zweiten Satzes an Registerparameterwerten eine zweite, von der ersten verschiedene, digitale Verarbeitungskette für zumindest einen Teil der Signalausgänge (7) der ersten Signalverarbeitungsstufe der zumindest einen Gruppe (20) von Pixelelementen (5) bereitgestellt wird.

14. Verfahren nach einem der Ansprüche 12 oder 13, wobei der erste und/oder zweite Satz an Registerparameterwerten in Abhängigkeit einer Röntgenanwendung bestimmt wird.

## Claims

1. Photon-counting X-ray detector (1) having a converter element (3) for converting X-rays into electric signals and a plurality of pixel elements (5), wherein
a. each pixel element (5) of the plurality of pixel elements (5) has a first signal processing stage for processing the electric signals in each case with at least one signal amplifier (17) and at least one comparator (19) for providing a digital pixel signal at a respective signal output (7) of the first signal processing stage of a pixel element (5) of the plurality of pixel elements (5),
b. the signal outputs (7) of the first signal processing stage of at least one group (20) of pixel elements (5) of the plurality of pixel elements (5) are coupled by means of signal technology to a common second signal processing stage having a multiplicity of digital logic elements (9) for digitally processing the digital pixel signals provided,
c. the common second signal processing stage has a configurable switching matrix (11) for interconnecting by means of signal technology at least one partial number of the multiplicity of digital logic elements (9) with the respective signal outputs (7) of the first signal processing stage of the group (20) of pixel elements (5) of the plurality of pixel elements (5),
wherein the multiplicity of digital logic elements (9) comprises at least one counting element (13) and one readout element (14),
wherein the at least one counting element (13) comprises a multiplicity of counters,
d. so that, following a configuration of the switching matrix (11), a processing chain can be provided for the digital processing of the digital pixel signals provided for each signal output (7) of the first signal processing stage of the group (20) of pixel elements (5) of the plurality of pixel elements (5),
and wherein the first signal processing stage is embodied by pixelwise pixel electronics so that a first signal processing stage, a corresponding pixel and sensor pixel electrode (16, 57) and a detection volume in the converter element (3) can be assigned to a pixel element (5) of the plurality of pixel elements (5) in each case, and the second signal processing stage can be assigned as a whole only to the group (20) of pixel elements (5).

2. Photon-counting X-ray detector (1) according to claim 1, wherein a first processing chain can be provided for a first number of the signal outputs (7) of the group (20) of pixel elements (5) and wherein a second processing chain different from the first processing chain can be provided for a second number of signal outputs (7).

3. Photon-counting X-ray detector (1) according to claim 1 or 2, wherein the multiplicity of digital logic elements (9) also comprises at least one element from the following list
- a coincidence logic,
- a signal-delay element,
- a buffer element,
- a switching element to prevent paralysis of a counting element,
- a combinational logic gate,
- a multiplexer,
- a register element,
- an element which, when triggered, creates a pulse of a fixed or configurable length,
- an element which measures the point in time or the duration of a trigger.

4. Photon-counting X-ray detector (1) according to one of claims 1 to 3, wherein a digital logic element (9) of the multiplicity of digital logic elements (9) is provided multiple times in the second signal processing stage.

5. Photon-counting X-ray detector (1) according to one of claims 1 to 4, wherein, following a configuration of the configurable switching matrix (11), non-interconnected digital logic elements (9) of the multiplicity of digital logic elements (9) are separated from a power supply.

6. Photon-counting X-ray detector (1) according to one of claims 1 to 5, wherein the plurality of pixel elements (5) is divided into a multiplicity of groups (20) of pixel elements (5), wherein each group (20) is in each case assigned to a configurable switching matrix (11).

7. Photon-counting X-ray detector (1) according to one of claims 1 to 5, wherein the plurality of pixel elements (5) is divided into a multiplicity of groups (20) of pixel elements, wherein a common configurable switching matrix (11) is assigned to the multiplicity of groups (20).

8. Photon-counting X-ray detector (1) according to claim 7, wherein the one configurable switching matrix (11) is configurable in groups.

9. Photon-counting X-ray detector (1) according to one of claims 1 to 8 having an evaluation unit (59) which provides the first processing stage and the second processing stage for at least one group (20) of pixel elements (5) and wherein the evaluation unit (59) provides at least one first region (61) for the first processing stage and one second region (63) for the second processing stage, wherein the at least one first region (61) is arranged in the form of an island within the second region (63) or on the edge along the second region (63).

10. Photon-counting X-ray detector (1) according to one of claims 1 to 8, having an evaluation unit (59) which provides the first processing stage and the second processing stage for at least one group (20) of pixel elements (5) and wherein the evaluation unit (59) has a layered structure and wherein the first processing stage is provided in a first layer (62) and the second processing stage is provided in a second layer (64) located after the first layer (62) in the direction of radiation incidence.

11. Medical imaging device having at least one photon-counting X-ray detector (1) according to one of the preceding claims.

12. Method for operating a photon-counting X-ray detector (1) embodied according to one of claims 1 to 10 for creating an X-ray image data set, wherein the at least one switching matrix (11) of the X-ray detector (1) can be configured by means of adjustable register parameters comprising the steps
a. adapting (S1) the adjustable register parameters based on a first set of register parameter values,
b. configuring (S2) the at least one configurable switching matrix (11) based on the adapted register parameters and thereby in each case providing a digital processing chain for at least one part of the signal outputs (7) of the first signal processing stage of the at least one group (20) of pixel elements (5) which is coupled to the at least one switching matrix (11) by means of signal technology,
c. receiving (S3) X-rays with the photon-counting X-ray detector (1) on the basis of which electric signals are created in the converter element (3),
d. processing (S4) the electric signals created by means of the first processing stage and the second processing stage of the photon-counting X-ray detector (1), wherein digital pixel signals are provided at a respective signal output (7) of the first signal processing stage of a pixel element (5) of the at least one group (20) of pixel elements (5) of the plurality of pixel elements (5) which are further processed by means of the digital processing chain provided in the second signal processing stage for a respective signal output (7),
e. outputting (S5) the processed digital pixel signals on the basis of which an X-ray image data set is created.

13. Method according to claim 12 , wherein in one step (S6) of the second adaptation, the adjustable register parameters are adapted based on a second set of register parameter values and wherein in one step (S7) of the second configuration of the at least one configurable switching matrix (11) by means of the second set of register parameter values, a second digital processing chain different from the first is provided for at least one part of the signal outputs (7) of the first signal processing stage of the at least one group (20) of pixel elements (5).

14. Method according to one of claims 12 or 13, wherein the first and/or second set of register parameter values is determined as a function of an X-ray application.

## Revendications

1. Détecteur (1) de rayons X comptant les photons comportant un élément (3) de convertisseur pour la transformation du rayonnement X en des signaux électriques et un nombre multiple d'éléments (5) de pixel,
dans lequel
a. chaque élément (5) de pixel du nombre multiple d'éléments (5) de pixel a un premier étage de traitement du signal pour le traitement des signaux électriques par respectivement au moins un amplificateur (17) de signal et par au moins un comparateur (19) pour disposer d'un signal de pixel numérique à une sortie (7) respective de signal du premier étage de traitement du signal d'un élément (5) de pixel du nombre multiple d'éléments (5) de pixel,
b. les sorties (7) de signal du premier étage de traitement du signal d'au moins un groupe (20) d'éléments (5) de pixel du nombre multiple d'éléments (5) de pixel sont reliés en technique du signal à un deuxième étage commun de traitement du signal, qui a une pluralité d'éléments (9) logiques numériques pour le traitement numérique des signaux de pixel numériques mis à disposition,
c. le deuxième étage commun du traitement du signal a une matrice (11) de commutation configurable pour la liaison en technique du signal d'au moins un nombre partiel de la pluralité d'éléments (9) logiques numériques aux sorties (7) de signal respectives du premier étage du traitement du signal du groupe (20) d'éléments (5) de pixel du nombre multiple d'éléments (5) de pixel,
dans lequel la pluralité d'éléments (9) logiques numériques comprend au moins un élément (13) de comptage et un élément (14) de lecture,
dans lequel le au moins un élément (13) de comptage comprend une pluralité de compteurs,
d. de manière à ce que, après une configuration de la matrice (11) de commutation pour chaque sortie (7) de signal du premier étage de traitement du signal du groupe (20) d'éléments (5) de pixel du nombre multiple d'éléments (5) de pixel, puisse être mise à disposition une chaîne de traitement pour le traitement numérique des signaux de pixel numériques mis à disposition,
et dans lequel le premier étage du traitement du signal est constitué par des électroniques de pixel à pixel, de manière à pouvoir affecter à un élément (5) de pixel du nombre multiple d'éléments (5) de pixel, respectivement un premier étage de traitement du signal, une électrode (16, 57) correspondante de pixel et de pixel de capteur et un volume de détection dans l'élément (3) de convertisseur, et le deuxième étage de traitement du traitement du signal peut être affecté dans son entier seulement au groupe (20) d'éléments (5) de pixel.

2. Détecteur (1) de rayons X comptant les photons suivant la revendication 1, dans lequel, pour un premier nombre des sorties (7) de signal du groupe (20) d'éléments (5) de pixel, il peut être mis à disposition une première chaîne de traitement et dans lequel, pour un deuxième nombre de sorties (7) de signal, il peut être mis à disposition une deuxième chaîne de traitement différente de la première chaîne de traitement.

3. Détecteur (1) de rayons X comptant les photons suivant la revendication 1 ou 2, dans lequel la pluralité d'éléments (9) logiques numériques comprend en outre au moins un élément de la liste suivante
- une logique de coïncidence,
- un élément de retard du signal,
- un élément tampon,
- un élément de commutation pour empêcher une paralyse d'un élément de comptage,
- un circuit logique combinatoire,
- un multiplexeur,
- un élément de repérage,
- un élément qui, au déclenchement, produit une impulsion d'une longueur fixe ou configurable,
- un élément, qui mesure l'instant ou la durée d'un déclencheur.

4. Détecteur (1) de rayons X comptant les photons suivant l'une des revendications 1 à 3, dans lequel un élément (9) logique numérique de la pluralité d'éléments (9) logiques numériques est mis à disposition plusieurs fois dans le deuxième étage du traitement du signal.

5. Détecteur (1) de rayons X comptant les photons suivant l'une des revendications 1 à 4, dans lequel, après une configuration de la matrice (11) de commutation configurable, des éléments (9) logiques numériques non reliés de la pluralité d'éléments (9) logiques numériques sont séparés d'une alimentation en puissance.

6. Détecteur (1) de rayons X comptant les photons suivant l'une des revendications 1 à 5, dans lequel le nombre multiple d'éléments (5) de pixel est subdivisé en une pluralité de groupes (20) d'éléments (5) de pixel, dans lequel chaque groupe (20) est affecté respectivement à une matrice (11) de commutation configurable.

7. Détecteur (1) de rayons X comptant les photons suivant l'une des revendications 1 à 5, dans lequel le nombre multiple d'éléments (5) de pixel est subdivisé en une pluralité de groupes (20) d'éléments de pixel, dans lequel à la pluralité de groupes (20) est affectée une matrice (11) de commutation commune configurable.

8. Détecteur (1) de rayons X comptant les photons suivant la revendication 7, dans lequel la une matrice (11) de commutation configurable peut être configurée groupe par groupe.

9. Détecteur (1) de rayons X comptant les photons suivant l'une des revendications 1 à 8, comportant une unité (59) d'évaluation, qui met à disposition le premier étage de traitement et le deuxième étage de traitement pour au moins un groupe (20) d'éléments (5) de pixel, et dans lequel l'unité (59) d'évaluation dispose d'au moins une première partie (61) pour le premier étage de traitement et d'une deuxième partie (63) pour le deuxième étage de traitement, dans lequel la au moins une première partie (61) est disposée en forme d'îlot au sein de la deuxième partie (63) ou du côté du bord le long de la deuxième partie (63).

10. Détecteur (1) de rayons X comptant les photons suivant l'une des revendications 1 à 8, comportant une unité (59) d'évaluation, qui met à disposition le premier étage de traitement et le deuxième étage de traitement pour au moins un groupe (20) d'éléments (5) de pixel, et dans lequel l'unité (59) d'évaluation est constituée couche par couche, et dans lequel le premier étage de traitement est mis à disposition dans une première couche (62) et le deuxième étage de traitement est mis à disposition dans une deuxième couche (64) disposée derrière la première couche (62) dans le sens d'incidence des rayons.

11. Appareil d'imagerie médicale comportant au moins un détecteur (1) de rayons X comptant les photons suivant l'une des revendications précédentes.

12. Procédé pour faire fonctionner un détecteur (1) de rayons X comptant les photons, qui est réalisé suivant l'une des revendications 1 à 10, pour la production d'un ensemble de données d'images de rayons X, dans lequel la au moins une matrice (11) de commutation du détecteur (1) de rayons X peut être configurée au moyen d'un paramètre de repérage réglable, comprenant les stades :
a. adaptation (S1) des paramètres de repérage réglables sur la base d'un premier ensemble de valeurs de paramètres de repérage,
b. configuration (S2) de la au moins une matrice (11) de commutation configurable sur la base des paramètres de repérages adaptés et mise à disposition ainsi respectivement d'une chaîne de traitement numérique pour au moins une partie des sorties (7) de signal du premier étage du traitement du signal du au moins un groupe (20) d'éléments (5) de pixel, qui sont reliés en technique du signal à la au moins une matrice (11) de commutation,
c. réception (S3) de rayonnement X, par le détecteur (1) de rayons X comptant les photons, des signaux électriques sur cette base étant produits dans l'élément (3) convertisseur,
d. traitement (S4) des signaux électriques produits au moyen du premier étage de traitement et du deuxième étage de traitement du détecteur (1) de rayons X comptant les photons, dans lequel on met à disposition des signaux de pixel numériques à une sortie (7) respective du signal du premier étage du traitement du signal d'un élément (5) de pixel du au moins un groupe (20) d'éléments (5) de pixel du nombre multiple d'éléments (5) de pixel, que l'on traite davantage dans le deuxième étage du traitement du signal au moyen de la chaîne numérique mise à disposition de la sortie (7) de signal respective,
e. émission (S5) des signaux de pixel numériques, traités sur la base desquels on produit un ensemble de données d'images de rayons X.

13. Procédé suivant la revendication 12, dans lequel, dans un stade (S6) de la deuxième adaptation, on adapte les paramètres de repérage réglables sur la base d'un deuxième ensemble de valeurs de paramètres de repérage et dans lequel, dans un stade (S7) de la deuxième configuration de la au moins une matrice (11) de commutation configurable, on met à disposition, au moyen du deuxième ensemble de valeurs de paramètres de repérage, une deuxième chaîne de traitement numérique, différente de la première, pour au moins une partie des sorties (7) du signal du premier étage du traitement du signal du au moins un groupe (20) d'éléments (5) de pixel.

14. Procédé suivant l'une des revendications 12 ou 13, dans lequel on détermine le premier et/ou le deuxième ensembles de valeurs de paramètres de repérage en fonction d'une application de rayons X.
